# EUROPEAN PATENT APPLICATION

(11) **EP 2 149 573 A1**
(43) Date of publication of application: **03.02.2010**
(21) Application number: 08380238.9
(22) Date of filing: 01.08.2008
(51) Int. Cl.: C07D 409/12, C07D 417/12, A61K 31/433, A61K 31/404, A61K 31/425, A61P 25/00

(54) **Substituted indole sulfonamide compounds their preparation and use as medicaments**

(71) Applicant: Laboratorios Del. Dr. Esteve, S.A., 08041 Barcelona (ES)
(72) Inventor: Beller, Matthias, 18211 Ostseebad Nienhagen (DE); Alex, Karolin, 18057 Rostock (DE)
(74) Representative: Carpintero Lopez, Francisco

(57) **Abstract**

The present invention relates to indole sulfonamide compounds of general formula (I), 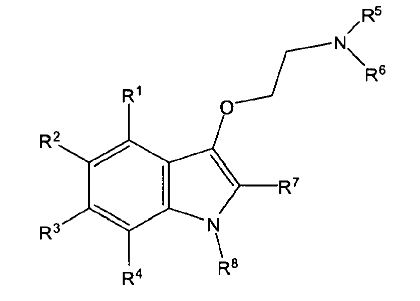 a process for their preparation, a medicament comprising these compounds and the use of the indole sulfonamide compounds for the preparation of medicaments for 5-HT₆ receptor regulation as well as for the treatment of disorders related thereto.

## Description

### FIELD OF THE INVENTION

The present invention relates to substituted indole sulfonamide compounds of general formula (I), 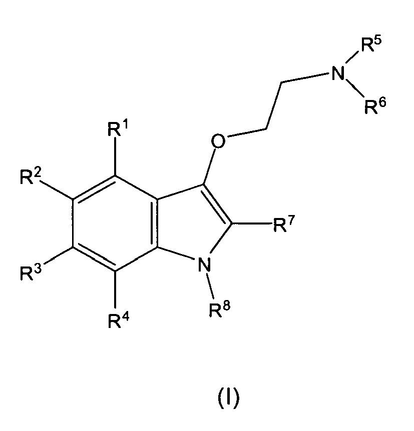 a process for their preparation, a medicament comprising these compounds and the use of substituted indole sulfonamide compounds for the preparation of medicaments for 5-HT₆ receptor regulation as well as for the treatment of disorders related thereto.

### BACKGROUND OF THE INVENTION

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F. J. Monsma, et al., Mol. Pharmacol., 1993, 43, 320; M. Ruat, et al., Biochem. Biophys. Res. Commun., 1993, 193, 268] and in humans [R. Kohen, et al., J. Neurochem., 1996, 66, 47]. Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka, et al., Ann. NY Acad. Sci., 1998, 861, 244; A. Bourson, et al., Br. J. Pharmacol., 1998, 125, 1562; D.C. Rogers, et al., Br. J. Pharmacol. Suppl., 1999, 127, 22P; A. Bourson, et al., J. Pharmacol. Exp. Ther., 1995, 274, 173; A.J. Sleight, et al., Behav. Brain Res., 1996, 73, 245; T. A. Branchek, et al., Annu. Rev. Pharmacol. Toxicol., 2000, 40, 319; C. Routledge, et al., Br. J. Pharmacol., 2000, 130, 1606]. It has been shown that typical and atypical antipsychotic drugs for treating schizophrenia have a high affinity for 5-HT₆ receptors [B. L. Roth, et al., J. Pharmacol. Exp. Ther., 1994, 268, 1403; C. E. Glatt, et al., Mol. Med., 1995, 1, 398; F. J. Mosma, et al., Mol. Pharmacol., 1993, 43, 320; T. Shinkai, et al., Am. J. Med. Genet., 1999, 88, 120]. Compounds with 5-HT₆ receptor affinity are useful for treating infant hyperkinesia (ADHD, attention deficit / hyperactivity disorder) [W. D. Hirst, et al., Br. J. Pharmacol., 2000, 130, 1597; C. Gérard, et al., Brain Research, 1997, 746, 207; M. R. Pranzatelli, Drugs of Today, 1997, 33, 379]. Moreover, it has been shown that the 5-HT₆ receptor also plays a role in food ingestion [Neuropharmacology, 2001, 41, 210-219]. Food ingestion disorders, particularly obesity, are a serious, fast growing threat to the health of humans of all age groups, since they increase the risk of developing other serious, even life-threatening diseases such as diabetes or coronary diseases.

Thus, it was an object of the present invention to provide novel compounds that are suitable in particular as active substances in medicaments, preferably in medicaments for the regulation of 5-HT₆ receptors, for cognitive enhancement, for the prophylaxis and/or treatment of food-intake related disorders, disorders of the central nervous system, disorders of the gastrointestinal tract, such as irritable intestine syndrome, anxiety, panic, depression, cognitive memory disorders, senile dementia disorders, such as Morbus Alzheimer, Morbus Parkinson and Morbus Huntington, schizophrenia, psychosis, infantile hyperkinesia, ADHD (attention deficit, hyperactivity disorders) and other 5-HT₆ mediated disorders particularly in mammals, including humans.

It has been found that the indole-derived sulfonamide compounds of general formula (I), given below show affinity for the 5-HT₆-receptor.

These compounds are therefore also suitable for the manufacture of a medicament for cognitive enhancement, for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-insulin Dependent Diabetes Mellitus), preferably type II diabetes, which is caused by obesity, disorders of the central nervous system, disorders of the gastrointestinal tract, such as irritable intestine syndrome, anxiety, panic, depression, cognitive memory disorders, senile dementia disorders, such as Morbus Alzheimer, Morbus Parkinson and Morbus Huntington, schizophrenia, psychosis, infantile hyperkinesia, ADHD (attention deficit, hyperactivity disorders) and other 5-HT₆ mediated disorders particularly in mammals, including man.

### DESCRIPTION OF THE INVENTION

Thus, in one of its aspects the present invention relates to an indole sulfonamide compound of general formula (I), 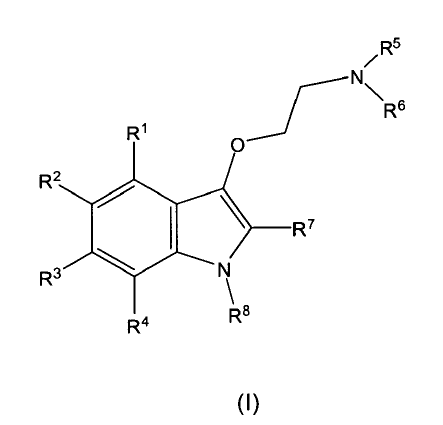 wherein
R¹, R², R³, and R⁴ independent from one another, each represents a hydrogen atom, an halogen atom, a C₁-C₆ alkyl radical, a C₁-C₆ alkoxy radical or a -N(R⁹)-S(=O)₂-R¹⁰ moiety
with the proviso that at least one of the substituents R¹, R², R³ and R⁴ represents a - N(R⁹)-S(=O)₂-R¹⁰ moiety
wherein
R⁹ represents a hydrogen atom or a C₁₋₁₀ alkyl radical optionally substituted with one or more substituents independently selected from C₁-C₆ alkyl, aryl, cyano, C₁-C₆ alkoxy and trifluoromethyl;
R¹⁰ represents aryl or heteroaryl optionally substituted with one or more substituents independently selected from -NO₂, -NH₂, -SH, -OH, -CN, -C(=O)-OH, -S(=O)₂-OH, -C(=O)-NH₂, -S(=O)₂-NH₂, -S(=O)₂-R¹¹ , -OR¹¹ , -SR¹¹, -C(=O)-OR¹¹ , -N(R¹¹)-S(=O)₂-R¹² , -NH-R" , -NR¹¹R¹² , -C(=O)-NHR¹¹, -C(=O)-NR¹¹R¹², -S(=O)₂-NHR¹¹, -S(=O)₂-NR¹¹R¹², -O-C(=O)-R¹¹, -NH-C(=O)-R¹¹ , -NR¹¹-C(=O)-R¹², -NH-C(=O)-O-R¹¹ , -NR¹¹-C(=O)-O-R¹² , -S(=O)₂-O-R¹¹, an halogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene, or an optionally at least mono-substituted aryl or heteroaryl radical, which may be bonded via a linear or branched alkylene group,
wherein R¹¹ and R¹², independent from one another, each represents a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R⁵ and R⁶, independent from one another, each represents a hydrogen atom; or a linear or branched, saturated or unsaturated, unsubstituted or at least mono-substituted aliphatic radical;
or
R⁵ and R⁶ together with the bridging nitrogen atom form a saturated or unsaturated, unsubstituted or at least mono-substituted, optionally at least one additional heteroatom as ring member containing heterocycloaliphatic radical, which may be condensed with an unsubstituted or at least mono-substituted saturated, unsaturated or aromatic mono- or bicyclic ring system;
or
R⁵ and R⁶ together with the bridging nitrogen atom form an unsubstituted or at least mono-substituted, optionally at least one additional heteroatom as ring member containing heteroaryl radical, which may be condensed with an unsubstituted or at least mono-substituted saturated, unsaturated or aromatic mono- or bicyclic ring system;
R⁷ represents a linear or branched, saturated or unsaturated, unsubstituted or at least mono-substituted aliphatic radical;
R⁸ represents a hydrogen atom; a -C(=O)-OR¹³ moiety; a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; or a benzyl moiety which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
R¹³ represents a linear or branched, saturated or unsaturated C₁₋₁₀ aliphatic radical
optionally in form of one of its stereoisomers, preferably enantiomers or diasteromers, its racemate or in the form of a mixture of at least two of its stereoisomers, preferably enantiomers or diastereomers, in any mixture ratio, or a salt, preferably a corresponding physiologically acceptable salt, or a prodrug, or a corresponding solvate.

If any of the substituent(s) represents or comprises a (hetero)cycloaliphatic radical, i.e. a cycloaliphatic radical or a heterocycloaliphatic, including a C₃₋₉ (hetero)cycloalkyl radical, i.e. a C₃₋₉ cycloalkyl or a C₃₋₉ heterocycloalkyl radical, or a C₄₋₉ (hetero)cycloalkenyl radical, i.e. a C₄₋₉ cycloalkenyl or a C₄₋₉ heterocycloalkenyl radical, said (hetero)cycloaliphatic radical, C₃₋₉ (hetero)cycloalkyl radical or C₄₋₉ (hetero)cycloalkenyl is - if not defined otherwise - unsubstituted or optionally substituted with one or more substituents, preferably unsubstituted or optionally substituted with 1, 2, 3, 4 or 5 substituent(s). Said substituent(s) may preferably be selected independently from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -O-CFH₂, -O-CF₂H, -S-CFH₂, -S-CF₂H, -SO₃H, -NH-C(=O)-C₁₋₅-alkyl, -N(C₁₋₅-alkyl)-C(=O)-C₁₋₅-alkyl, -C(=O)-C₁₋₅-perfluoroalkyl, -S(=O)₂-NH₂, -S(=O)₂-NH-C₁₋₅-alkyl, -S(=O)₂-NH-phenyl, -NH-S(=O)₂-C₁₋₅-alkyl, cyclobutyl, cyclopropyl, cyclohexyl, cyclopentyl, cyclohexyl, pyrrolidinyl, piperidinyl, morpholinyl, phenyl, phenethyl, phenoxy and benzyl; whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituent(s) may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenethyl, phenoxy and benzyl; whereby in each occurrence the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

If any of the substituent(s) in any of the herein defined formulae represents or comprises a (hetero)cycloaliphatic radical, i.e. a cycloaliphatic or a heterocycloaliphatic radical, including a C₃₋₉ (hetero)cycloalkyl radical, i.e. a C₃₋₉ cycloalkyl radical or a C₃₋₉ heterocycloalkyl radical, or a C₄₋₉ (hetero)cycloalkenyl, i.e. a C₄₋₉ cycloalkenyl or a C₄₋₉ heterocycloalkenyl radical, which contains one or more, preferably 1, 2 or 3 heteroatom(s) as ring member(s), unless defined otherwise, each of these heteroatom(s) may preferably be selected independently from the group consisting of nitrogen, oxygen and sulfur.

If any of the substituent(s) in any of the herein defined formulae represents or comprises a C₃₋₉ (hetero)cycloalkyl radical, i.e. a C₃₋₉ cycloalkyl or a C₃₋₉ heterocycloalkyl radical, or a C₄₋₉ (hetero)cycloalkenyl, i.e a C₄₋₉ cycloalkenyl or a C₄₋₉ heterocycloalkenyl radical, which contains optionally 1 or 2 additional heteroatom(s) as ring member(s), unless defined otherwise, each of these heteroatom(s) may preferably be selected independently from the group consisting of nitrogen, oxygen and sulfur.

Suitable saturated or unsaturated, optionally at least one heteroatom as ring member containing (hetero)cycloaliphatic radicals, i.e. cycloaliphatic or heterocycloaliphatic radicals, C₃₋₉ (hetero)cycloalkyl radicals, i.e. C₃₋₉ cycloalkyl or C₃₋₉ heterocycloalkyl radicals or C₄₋₉ (hetero)cycloalkenyl radicals, i.e. C₄₋₉ cycloalkenyl or C₄₋₉ heterocycloalkenyl radicals may preferably be selected from the group consisting of cyclopropyl, cyclobutyl, cyclopentyl, cyclohexyl, cycloheptyl, cyclooctyl, cyclononyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, 2,5-dihydro-1H-tetrazolyl, (2,3)-dihydro-1H-pyrrolyl, (4,5)-dihydro-1H-pyrazolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, octahydrocyclopenta[c]pyrrolyl, (1,3)-dioxanyl, (1,4)-dioxanyl and (1,3)-dioxolanyl.

Suitable saturated or unsaturated, optionally at least one heteroatom as ring member containing (hetero)cycloaliphatic radicals, i.e. cycloaliphatic or heterocycloaliphatic radicals, C₃₋₉ (hetero)cycloalkyl radicals, i.e. C₃₋₉ cycloalkyl or C₃₋₉ heterocycloalkyl radicals or C₄₋₉ (hetero)cycloalkenyl radicals, i.e. C₄₋₉ cycloalkenyl or C₄₋₉ heterocycloalkenyl radicals, which are condensed with an unsubstituted or at least mono-substituted mono- or bicyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydrocarbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, octahydropyrrolo[1,2-a]pyrazinyl, octahydro-2H-pyrido[3,2-b][1,4]oxazinyl, octahydro-1H-cyclopenta[b]pyridinyl, dodecahydro-1H-carbazolyl, octahydrofuro[2,3-b]pyridinyl, fluorenyl, carbazolyl, (4,5,6,7)-tetrahydro-1H-indolyl, octahydro-1H-pyrido[1,2-α]pyrazine, (3a,4,5,6,7,7a)-hexahydro-1H-pyrrolo[2,3-b]pyridinyl, (3a,4,5,6,7,7a)-hexahydrothieno[2,3-b]pyridinyl, (3a,4,5,6,7,7a)-hexahydrofuro[2,3-b]pyridinyl, octahydrothieno[2,3-b]pyridinyl, octahydro-1H-pyrazino[1,2-a]pyrimidinyl, octahydro-1H-pyrrolo[2,3-b]pyridinyl, octahydropyrrolo[1,2-α]pyrazinyl, octahydro-1H-indolyl, octahydrocyclopenta[b]pyrrolyl and (1,2,3,4)-tetrahydroquinoxazlinyl.

Suitable saturated or unsaturated C₃₋₉ heterocycloalkyl and C₄₋₉ heterocycloalkenyl rings may preferably be selected from the group consisting of pyrrolidinyl, piperidinyl, piperazinyl, homopiperazinyl, morpholinyl, aziridinyl, azetidinyl, imidazolidinyl, thiomorpholinyl, pyrazolidinyl, azepanyl, diazepanyl, azocanyl, (1,2,3,6)-tetrahydropyridinyl, (1,2,3,4)-tetrahydropyridinyl, (1,2,5,6)-tetrahydropyridinyl, hexahydropyrimidinyl, (5,6)-dihydro-4H-pyrimidinyl and (5,6)-dihydro-4H-pyrimidinyl.

Suitable saturated or unsaturated C₃₋₉ heterocycloalkyl and C₄₋₉ heterocycloalkenyl rings, which are condensed with an unsubstituted or at least mono-substituted mono- or bicyclic ring system may preferably be selected from the group consisting of indolinyl, isoindolinyl, (1,2,3,4)-tetrahydroquinolinyl, (1,2,3,4)-tetrahydroisoquinolinyl, octahydro-cyclopenta[c]pyrrolyl, decahydroquinolinyl, dodecahydrocarbazolyl, 9H-carbazolyl, decahydroisoquinolinyl, (2,3)-dihydro-1 H-cyclopenta[b]indolyl, [1,2,3,4]-tetrahydroquinazolinyl, [3,4]-dihydro-2H-benzo[1,4]oxazinyl, octahydropyrrolo[1,2-a]pyrazinyl, octahydro-2H-pyrido[3,2-b][1,4]oxazinyl, octahydro-1H-cyclopenta[b]pyridinyl, dodecahydro-1H-carbazolyl, octahydrofuro[2,3-b]pyridinyl, carbazolyl, (4,5,6,7)-tetrahydro-1H-indolyl, octahydro-1H-pyrido[1,2-α]pyrazine, (3a,4,5,6,7,7a)-hexahydro-1H-pyrrolo[2,3-b]pyridinyl, (3a,4,5,6,7,7a)-hexahydrothieno[2,3-b]pyridinyl, (3a,4,5,6,7,7a)-hexahydrofuro[2,3-b]pyridinyl, octahydrothieno[2,3-b]pyridinyl, octahydro-1H-pyrazino[1,2-a]pyrimidinyl, octahydro-1H-pyrrolo[2,3-b]pyridinyl, octahydro-1H-indolyl, octahydropyrrolo[1,2-α]pyrazinyl, octahydrocyclopenta[b]pyrrolyl and (4,5,6,7)-tetrahydro-1 H-indolyl.

If any of the substituents in any of the herein defined formulae represents an alkylene group, including an C₁₋₆ alkylene group, an alkenylene group, including an C₂₋₆ alkenylene group or an alkinylene group, including an C₂₋₆ alkinylene group, said alkylene group, C₂₋₆ alkylene group, alkenylene group, C₂₋₆ alkenylene group, alkinylene group or C₂₋₆ alkinylene group is unsubstituted or substituted with one or more substituents, preferably unsubstituted or optionally substituted with 1, 2, 3, 4 or 5 substituent(s). Said substituent(s) may preferably be selected independently from the group consisting of -O-C₁₋₅-alkyl, -S-C₁₋₅-alkyl, -F, Cl, Br, I, -CN, -CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. An alkenylene group comprises at least one carbon-carbon double bond, an alkinylene group comprises at least one carbon-carbon triple bond.

Suitable alkylene groups include -(CH₂)-, -CH(CH₃)-, -CH(phenyl), -(CH₂)₂-, -(CH₂)₃-, -(CH₂)₄-, -(CH₂)₅ and -(CH₂)₆-, suitable alkenylene groups include -CH=CH-, -CH₂-CH=CH- and -CH=CH-CH₂- and suitable alkinylene groups include -C≡C-, -CH₂-C≡C- and -C≡C-CH₂-.

If any of the substituents represents or comprises an aryl radical, including a 6-membered aryl radical such as phenyl or a 10-membered aryl radical such as naphthyl or a 14-membered aryl radical such as anthracenyl, said aryl radical is - if not defined otherwise - unsubstituted or substituted with one or more substituents, preferably unsubstituted or optionally substituted with 1, 2, 3, 4 or 5 substituent(s). Said substituent(s) - if not defined otherwise - may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -O-CFH₂, -O-CF₂H, -S-CFH₂, -S-CF₂H, -SO₃H, -NH-C(=O)-C₁₋₅-alkyl, -N(C₁₋₅-alkyl)-C(=O)-C₁₋₅-alkyl, -C(=O)-C₁₋₅-perfluoroalkyl, -S(=O)₂-NH₂, -S(=O)₂-NH-C₁₋₅-alkyl, -S(=O)₂-NH-phenyl, -NH-S(=O)₂-C₁₋₅-alkyl, cyclobutyl, cyclopropyl, cyclohexyl, cyclopentyl, cyclohexyl, pyrrolidinyl, piperidinyl, morpholinyl, phenyl, phenethyl, phenoxy and benzyl; whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

Preferred aryl radicals, which may optionally be at least mono-substituted, are phenyl and naphthyl.

If any of the substituents in any of the herein defined formulae represents or comprises a heteroaryl radical, including a monocyclic 5- or 6-membered heteroaryl radical or a bi- or tricyclic 8-, 9-, 10-, 11-, 12-, 13- or 14- membered heteroaryl radical, said heteroaryl radical is - if not defined otherwise - unsubstituted or substituted by one or more substituents, preferably unsubstituted or optionally substituted with 1, 2, 3, 4 or 5 substituent(s). Said substituent(s) - if not defined otherwise - may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -O-CFH₂, -O-CF₂H, -S-CFH₂, -S-CF₂H, -SO₃H, -NH-C(=O)-C₁₋₅-alkyl, -N(C₁₋₅-alkyl)-C(=O)-C₁₋₅-alkyl, -C(=O)-C₁₋₅-pertluoroalkyl, -S(=O)₂-NH₂, -S(=O)₂-NH-C₁₋₅-alkyl, -S(=O)₂-NH-phenyl, -NH-S(=O)₂-C₁₋₅-alkyl, cyclobutyl, cyclopropyl, cyclohexyl, cyclopentyl, cyclohexyl, pyrrolidinyl, piperidinyl, morpholinyl, phenyl, phenethyl, phenoxy and benzyl; whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2, 3 or 4 heteroatom(s).

Suitable bi- or tricyclic heteroaryl radicals, which may optionally be at least mono-substituted, may preferably be selected from the group consisting of indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl.

Suitable mono-, bi- or tricyclic heteroaryl radicals, which are condensed with an unsubstituted or at least mono-substituted saturated or unsaturated mono- or bicyclic ring system, may preferably be selected from the group consisting of (4,5,6,7)-tetrahydro-1H-indolyl, [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl.

Suitable monocyclic 5- or 6-membered heteroaryl radicals, which may optionally be at least mono-substituted, may preferably be selected from the group consisting of pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl.

A mono- or bicyclic ring system according to the present invention - if not defined otherwise - means a mono- or bicyclic hydrocarbon ring system that may be saturated, unsaturated or aromatic. Each of its rings may show a different degree of saturation, i.e. it may be saturated, unsaturated or aromatic. Optionally each of the rings may contain one or more, preferably 1, 2 or 3 heteroatom(s) as ring member(s), which may be independent from one another and which can preferably be selected from the group consisting of nitrogen, oxygen and sulphur. The rings of the mono- or bicyclic ring system are preferably 5-, 6- or 7- membered. Examples of mono- and bicyclic rings are phenyl or naphtyl ring systems.

The term "condensed" according to the present invention means that a ring or ring system is attached to another ring or ring system, whereby the terms "annulated" or "annelated" are also used by those skilled in the art to designate this kind of attachment.

Such a mono- or bicyclic ring system is - if not defined otherwise - unsubstituted or substituted by one or more substituents, preferably unsubstituted or optionally substituted with 1, 2, 3, 4 or 5 substituent(s). Said substituents may preferably be selected independently from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -O-CFH₂, -O-CF₂H, -S-CFH₂, -S-CF₂H, -SO₃H, -NH-C(=O)-C₁₋₅-alkyl, -N(C₁₋₅-alkyl)-C(=O)-C₁₋₅-alkyl, -C(=O)-C₁₋₅-perfluoroalkyl, -S(=O)₂-NH₂, -S(=O)₂-NH-C₁₋₅-alkyl, -S(=O)₂-NH-phenyl, -NH-S(=O)₂-C₁₋₅-alkyl, cyclobutyl, cyclopropyl, cyclohexyl, cyclopentyl, cyclohexyl, pyrrolidinyl, piperidinyl, morpholinyl, phenyl, phenethyl, phenoxy and benzyl; whereby in each occurrence C₁₋₅-alkyl may be linear or branched and whereby the cyclic part(s) of said substituent(s) is unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenethyl, phenoxy and benzyl; whereby in each occurrence the cyclic part(s) of said substituent(s) is unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

If any of the substituents in any of the herein defined formulae represents a saturated or unsaturated aliphatic radical, i.e. an alkyl radical, including an C₁₋₁₀ alkyl radical; an alkenyl radical, including an C₂₋₁₀ alkenyl radical or an alkinyl radical, including an C₂₋₁₀ alkinyl radical; said aliphatic radical is - if not defined otherwise - unsubstituted or substituted with one or more substituents, preferably unsubstituted or optionally substituted with 1, 2, 3, 4 or 5 substituent(s). Said substituent(s) may preferably - if not defined otherwise - be selected independently from the group consisting of -O-C₁₋₅-alkyl, -S-C₁₋₅-alkyl, F, Cl, Br, I, -CN, -CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. More preferably said substituent(s) may preferably be selected independently from the group consisting of -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, NH-CH₃, -NH-C₂H₅, -NH-CH₂-CH₂-CH₃, -NH-CH(CH₃)₂, -NH-C(CH₃)₃, -N(CH₃)₂ and -N(C₂H₅)₂.

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, neo-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

In one embodiment R² is preferably -N(R⁹)-S(=O)₂-R¹⁰. It is also preferred that R⁹ is hydrogen, or C₁₋₁₀ alkyl optionally substituted with an aryl group, and more preferably R⁹ is hydrogen, or C₆ alkyl, or benzyl. Also preferred is that R¹⁰ represents an heteroaryl radical, which is a 8- or 9- membered aromatic bicyclic system, having from 1 to 3 heteroatoms independently selected from nitrogen and sulphur atoms, more preferred are benzothiadiazole, benzothiophene, imidazothiazole and benzothiophene bicyclic systems, and optionally, said bicyclic systems are substituted with one or more halogen atoms, preferably chloro, and/or with a linear saturated aliphatic radical, preferably methyl. Moreover it is also preferred that R¹, R³, and R⁴ are hydrogen. Further it is preferred that R⁷ is a saturated aliphatic radical; more preferably methyl. Moreover it is also preferred that R⁸ is hydrogen, or a -C(=O)-OR¹³ moiety or an optionally substituted saturated C₁₋₁₀ aliphatic radical; more preferably methyl, or benzyl. And R¹³ is preferably a branched saturated C₁₋₁₀ aliphatic radical, more preferably t-butyl. Further it is also preferred that R⁵ and R⁶, independent from one another, each represents a hydrogen atom; or a linear or branched, saturated or unsaturated, unsubstituted or at least mono-substituted aliphatic radical; more preferably R⁵ and R⁶ are the same, i.e. a saturated C₁₋₁₀ alkyl radical, most preferably ethyl.

Most preferred compounds falling under formula (I) include the following:
**(1)** 2,1,3-Benzothiadiazole-5-sulfonic acid [3-(2-diethylamino-ethoxy)-1,2-dimethyl-1*H*-indol-5-yl]-amide **(5a)**
**(2)** 2,1,3-Benzothiadiazole-4-sulfonic acid [1-benzyl-3-(2-diethyl-aminoethoxy)-2-methyl-1*H*-indol-5-yl]-amide **(5b)**
**(3)** 5-[(2,1,3-Benzothiadiazole-4-sulfonyl)-hexyl-amino]-3-(2-dieth-ylamino-ethoxy)-2-methyl-indole-1-carboxylic acid *tert*-butyl ester **(5c)**
**(4)** 5-Chloro-3-methyl-benzo[*b*]thiophene-2-sulfonic acid [3-(2-diethylamino-ethoxy)-1,2-dimethyl-1*H*-indol-5-yl]-amide (5d)
**(5)** 5-[(6-Chloro-imidazo[2,1-*b*]thiazole-5-sulfonyl)-hexyl-amino]-3-(2-diethylamino-ethoxy)-2-methyl-indole-1-carboxylic acid *tert*-butyl ester **(5e)**
**(6)** Benzo[*b*]thiophene-2-sulfonic acid [3-(2-diethylamino-ethoxy)-1,2-dimethyl-1*H-*indol-5-yl]-amide **(5f)**
**(7)** 5-[(Benzo[*b*]thiophene-2-sulfonyl)-benzyl-amino]-3-(2-diethyl-amino-ethoxy)-2-methyl-indole-1-carboxylic acid *tert*-butyl ester **(5g)**
**(8)** 5-(6-Chloro-imidazo[2,1-*b*]thiazole-5-sulfonylamino)-3-(2-diethylamino-ethoxy)-2-methyl-indole-1-carboxylic acid tert-butyl ester **(5h)**
**(9)** Benzo[*b*]thiophene-3-sulfonic acid benzyl-[3-(2-diethylamino-ethoxy)-2-methyl-1*H*-indol-5-yl]-amide **(6a)**
**(10)** 6-Chloro-imidazo[2,1-b]thiazole-5-sulfonic acid [3-(2-diethylamino-ethoxy)-2-methyl-1H-indol-5-yl]-amide **(6b)**

These compounds are shown in the following **Table 1**, in column 4 under **"product",** and referenced in column 5 under "**5** and **6**".

**Table 1**

| **Entry** | **Starting material** | **Sulfonyl-chloride** | **Product** | **5 and 6** |
|---|---|---|---|---|
| **(1)** | **4a** | 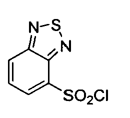 | 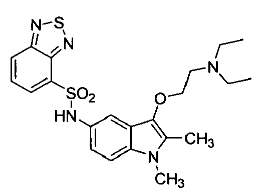 | **5a** |
| **(2)** | **4b** | 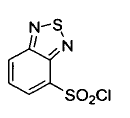 | 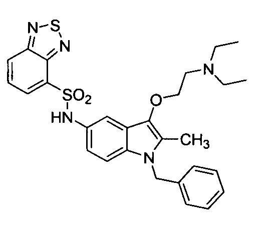 | **5b** |
| **(3)** | **3e** | 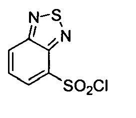 | 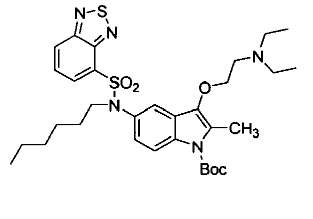 | **5c** |
| **(4)** | **4a** | 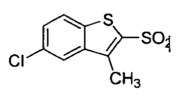 | 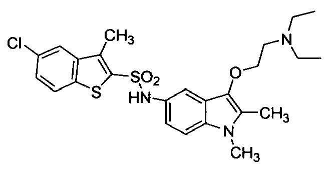 | **5d** |
| **(5)** | **3e** | 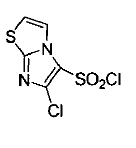 | 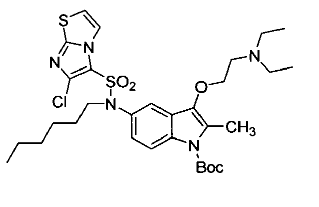 | **5e** |
| **(6)** | **4a** | 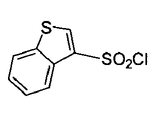 | 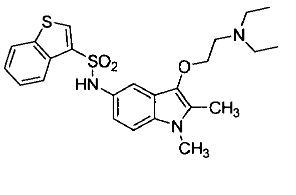 | **5f** |
| **(7)** | **3c** | 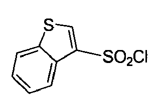 | | **5g** |
| **(8)** | **5g** | | 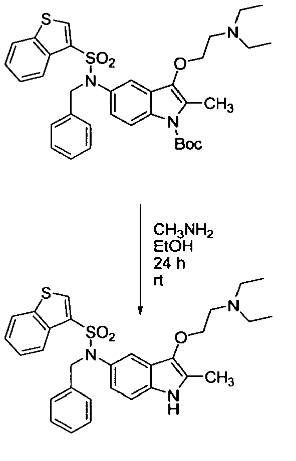 | **6a** |
| **(9)** | **4c** | 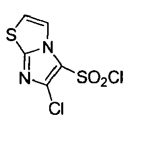 | 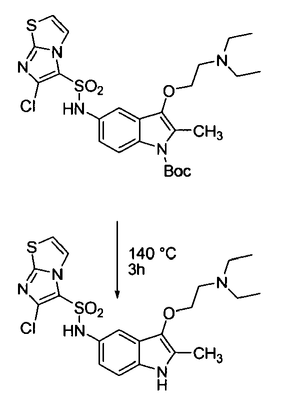 | **5h** |
| **(10)** | **5h** | | | **6b** |

In another aspect the invention relates to a process for the preparation of an indole sulphonamide compound of general formula (I), wherein at least one compound of formula **3** or **4** 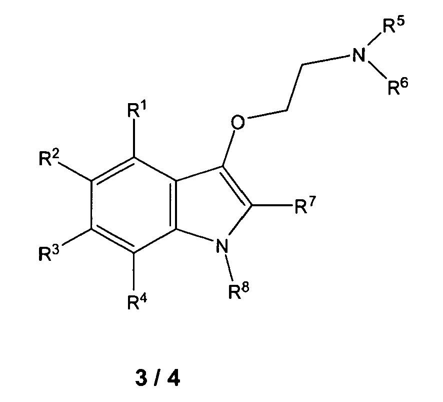 wherein R¹, R², R³, R⁴, R⁵, R⁶, R⁷ and R⁸ have the meaning as defined above, with the proviso that at least one of R¹, R², R³, R⁴ is -NHR⁹ in compound of formula **3**, or -NH₂ in compound of formula **4**, is reacted with at least one compound of general formula **(II),**

**R¹⁰SO₂X** **II,**

wherein R¹⁰ has the respective meaning as defined above and **X** represents a leaving group preferably selected from the group consisting of Cl and Br, optionally in the presence of at least one base, preferably in the presence of at least one inorganic base selected from the group consisting of alkali metal hydroxides or carbonates or in the presence of at least one organic base selected from the group consisting of triethylamine or pyridine, at temperatures from 0°C to room temperature and with reaction time from 5 minutes to 24 hours to yield at least one compound of general formula (I), 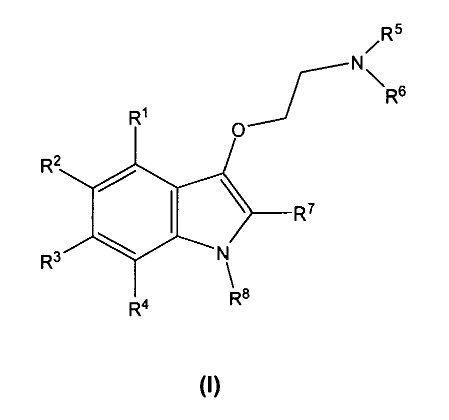 Wherein R¹, R², R³, R⁴, R⁵, R⁶, R⁷ and R⁸ have the meaning as defined above, with the proviso that at least one of R¹, R², R³, R⁴ is -N(R⁹)SO₂R¹⁰.

Preferably, the aforementioned reaction is carried out in an organic solvent, preferably an alkyl ether, more preferably diethyl ether, a cycloalkyl ether, more preferablly tetrahidrofurane or dioxane, an halogenated organic hydrocarbon, more preferably methylene chloride or chloroform, an alcohol, more preferably methanol or ethanol, an aprotic dipolar solvent, more preferably acetonitrile, pyridine or dimethylformamide.

As it can be seen from Table 1, treatment of the respective 5-aminoindole compounds (4a, 4b, 4c, 3c and 3e) with the different aryl- and heteroarylsulfonylchlorides shown in column 3 in Et₃N at 40 °C or in CH₂Cl₂ in the presence of Cs₂CO₃ at room temperature for 2 hours gave the corresponding sulfonylated indole derivatives **5a-g** in general good yields (Table 1).

The process comprises further, the preparation of a compound of formulae **3** or **4,** which is obtained according to the following synthetic route which comprises:
**a)** deprotecting at least one compound of formula (**1**) 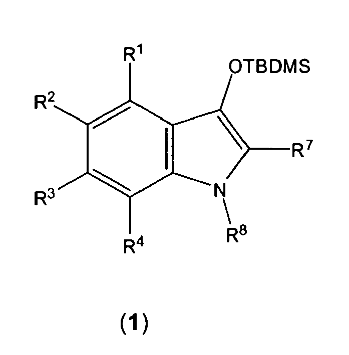 wherein R¹, R², R³, R⁴, R⁷ and R⁸ have the meaning as defined above, with the proviso that at least one of R¹, R², R³, R⁴ is Br, with a desilylating agent as TBAF at room temperature in a reaction medium, such as THF, followed by condensation with at least one compound of general formula **(III)**

   **R⁵R⁶NCH₂CH₂X** **III**

   wherein R⁵ and R⁶ have the meaning as defined above, and **X** represents a leaving group preferably selected from the group consisting of Cl and Br, at temperatures from 25 to 100 °C with reaction time from 3 to 12 hours, to yield at least one compound of formula **2** 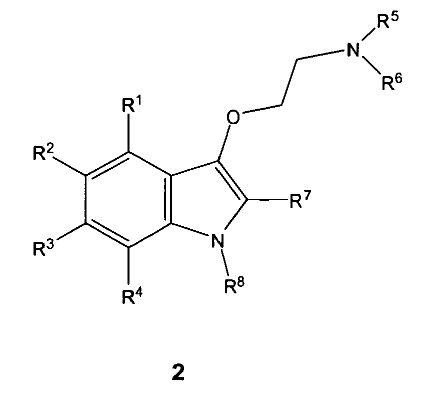 wherein R¹, R², R³, R⁴, R⁵ and R⁶, R⁷ and R⁸ have the meaning as defined above;
**b)** palladium-catalyzed amination of at least a compound of formula **2** with a compound of formula **(IV)**

   **NH₂R⁹** **(IV)**

   to yield at least a compound of formula **3** wherein R⁹ has the meaning as defined above; and
**c)** optionally catalyzed hydrogenation of at least a compound of formula **3** to yield at least a compound of formula **4**.

Best results for the palladium-catalyzed amination are obtained applying Pd(OAc)₂/*N*-phenyl-2-(di-1-adamantylphosphino)pyrrole as in situ catalyst at 100 °C for 20 h in toluene in attendance of LiHMDS (or Cs₂CO₃) as base.
The catalyzed hydrogenation is preferably carried out with H₂ over Pd/C.

The general process for the preparation of compounds of formula (I) as described above, is also depicted in general scheme I:

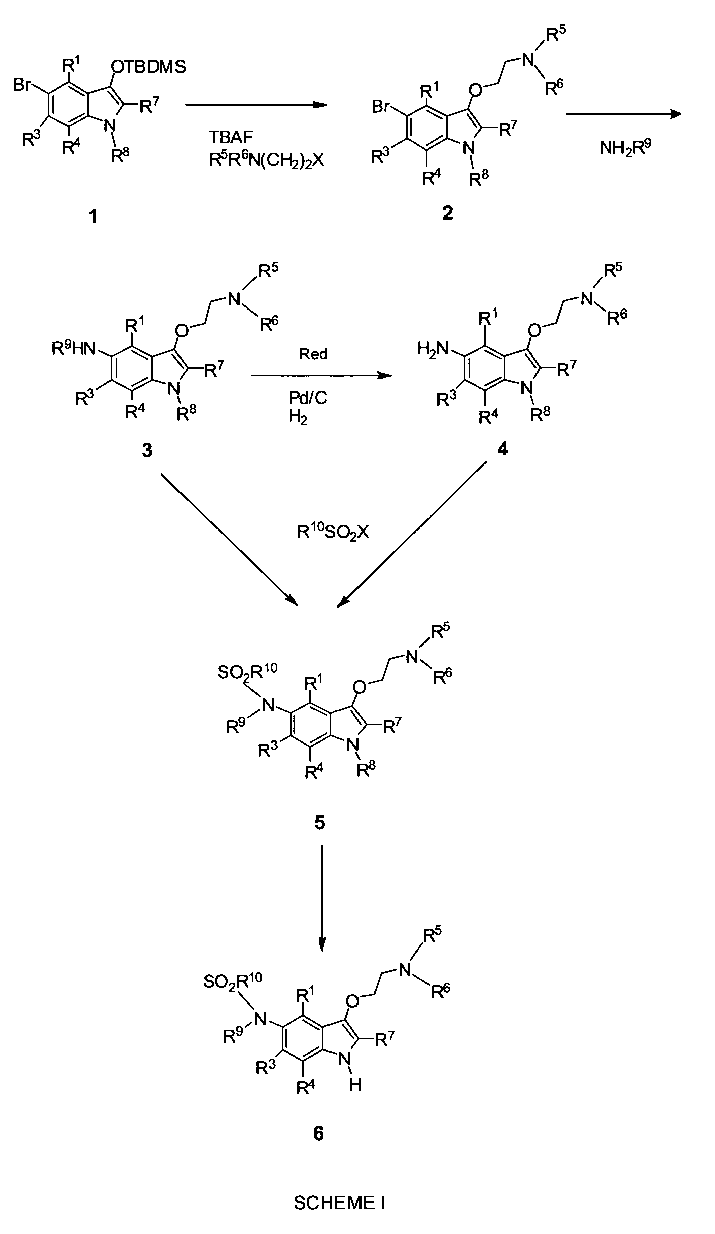

Further a compound of formula (I) wherein R⁸ is a radical as above defined, different from hydrogen, may be deprotected to yield the corresponding compound of formula (I) wherein R⁸ is hydrogen according to known method in the art. In this sense, the receptor-ligand affinity increases additionally with a free NH-group in position 1. Thus, inventors have deprotected compound **5g** and **5h** to the biologically more active indole derivatives **6a** and **6b** which are obtained in very good yields up to 88 % (Table 1).

The compounds of general formula (I), given above may be purified and/or isolated according to methods well known to those skilled in the art. Preferably, the compounds of general formula (I), may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

During some synthetic reactions described above or while preparing the compounds of general formula (I), the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted indole sulfonamide compounds of general formula (I), are obtained in form of a mixture of stereoisomers, particularly enantiomers or diastereomers, said mixtures may be separated by standard procedures known to those skilled in the art, e.g. chromatographic methods or crystallization with chiral reagents.

The substituted indole sulfonamide compounds of general formula (I), and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above. Suitable inorganic acids include but are not limited to hydrochloric acid, hydrobromic acid, phosphoric acid, oxalic acid, sulfuric acid, nitric acid, suitable organic acids include but are not limited to citric acid, maleic acid, fumaric acid, tartaric acid, or derivatives thereof, p-toluenesulfonic acid, methanesulfonic acid or camphersulfonic acid.

The term "salt" is to be understood as meaning any form of the substituted indole sulfonamide compounds in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

Solvates, preferably hydrates, of the substituted indole sulfonamide compounds of general formula (I), and in each case of corresponding stereoisomers may also be obtained by standard procedures known to those skilled in the art.

The term "solvate" according to this invention is to be understood as meaning any form of the substituted indole sulfonamide compounds in which they have attached to it via non-covalent binding another molecule (most likely a polar solvent) especially including hydrates and alcoholates, e.g. methanolate.

The term "prodrug" according to this invention is to be understood in its broadest sense encompassing those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

In another aspect the invention relates to new intermediate compounds of the following formulae **3** and **4** which are useful in the preparation of an indole sulphonamide compound of general formula (I), 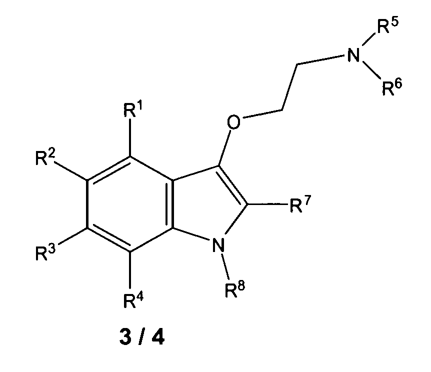 wherein R¹, R², R³, R⁴, R⁵, R⁶, R⁷ and R⁸ have the meaning as defined above, with the proviso that at least one of R¹, R², R³, R⁴ is -NHR⁹ in compound of formula **3**, or -NH₂ in compound of formula **4**, and wherein R⁹ has the meaning as defined above.

In a particular embodiment compounds falling within formulae **3** and **4** are the following:
Benzyl-[3-(2-diethylamino-ethoxy)-1,2-dimethyl-1*H*-indol-5-yl]-amine **(3a)**
Benzyl-[1-benzyl-3-(2-diethylamino-ethoxy)-2-methyl-1*H*-indol-5-yl]-amine **(3b)**
5-Benzylamino-3-(2-diethylamino-ethoxy)-2-methyl-indole-1-carboxylic acid *tert-*butyl ester **(3c)**
[3-(2-Diethylamino-ethoxy)-1,2-dimethyl-1*H*-indol-5-yl]-hexyl-amine **(3d)**
3-(2-Diethylamino-ethoxy)-5-hexylamino-2-methyl-indole-1-carboxylic acid *tert*-butyl ester **(3e)**
[3-(2-Diethylamino-ethoxy)-1,2-dimethyl-1*H*-indol-5-yl]-[2-(4-fluoro-phenyl)-ethyl]-amine **(3f)**
3-(2-Diethylamino-ethoxy)-1,2-dimethyl-1*H*-indol-5-ylamine **(4a)**
1-Benzyl-3-(2-diethylamino-ethoxy)-2-methyl-1*H*-indol-5-ylamine **(4b)**
5-Amino-3-(2-diethylamino-ethoxy)-2-methyl-indole-1-carboxylic acid *tert*-butyl ester **(4c)**

In another aspect the invention is directed to a medicament which comprises at least a one indole sulphonamide compound of general formula (I), optionally in form of one of its stereoisomers, preferably enantiomers or diasteromers, its racemate or in form of a mixture of at least two of its stereoisomers, preferably enantiomers or diastereomers, in any mixture ratio, or a salt, preferably a physiologically acceptable salt, or a prodrug, or a corresponding solvate and at least a pharmaceutical acceptable carrier, adjuvant or vehicle.

Said medicament is particularly suitable for 5-HT₆ receptor regulation and therefore for the treatment and/or prophylaxis of diseases or disorders that are at least partially mediated via 5-HT₆ receptors.

In a preferred embodiment said medicament is suitable for the prophylaxis and/or treatment of food intake disorders, preferably for the regulation of appetite, for the maintenance, increase or reduction of body weight, for the prophylaxis and/or treatment of obesity, for the prophylaxis and/or treatment of bulimia, for the prophylaxis and/or treatment of anorexia, for the prophylaxis and/or treatment of cachexia, for the prophylaxis and/or treatment of type II diabetes (non-insulin dependent diabetes mellitus); for the prophylaxis and/or treatment of cognitive disorders, preferably memory disorders; or for improvement of cognition (for cognitive enhancement).

In another preferred embodiment said medicament is suitable for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia or for the prophylaxis and/or treatment hyperactivity disorder (ADHD, attention deficit, hyperactivity disorder).

The medicament obtained according to the present invention is particularly suitable for the administration to mammals, including man. The drug can preferably be administered to all age groups, namely, children, adolescents and adults.

Another aspect of the present invention is the use of at least one indole sulfonamide compound of general formula (I) , optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate, for the manufacture of a drug for regulating the 5-HT₆ receptor, for the prophylaxis and/or treatment of food intake disorders, preferably for the regulation of appetite, for the maintenance, increase or reduction of body weight, for the prophylaxis and/or treatment of obesity, for the prophylaxis and/or treatment of bulimia, for the prophylaxis and/or treatment of anorexia, for the prophylaxis and/or treatment of cachexia, for the prophylaxis and/or treatment of type II diabetes (non-insulin dependent diabetes mellitus); for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment cognitive disorders, preferably memory disorders; for improvement of cognition (for cognitive enhancement); for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia or for the prophylaxis and/or treatment hyperactivity disorder (ADHD, attention deficit, hyperactivity disorder).

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parenterally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective indole sulfonamide compound is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more substituted indole sulfonamide compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from 0.01 to 4000 mg, preferably 0.1 to 2000 mg, more preferably 0.5 to 1000 of active substance to be administered during one or several intakes per day.

The following examples are given only as further illustration of the present invention, and they should not be taken as a definition of the limits of the invention.

### EXAMPLES

All reactions were carried out under an argon atmosphere. Chemicals were purchased from Aldrich, Fluka, Acros and Strem and unless otherwise noted were used without further purification. All compounds were characterized by ¹H NMR, ¹³C NMR, MS, HRMS and IR spectroscopy. ¹H and ¹³C NMR spectra were recorded on Bruker AV 300, AV 400 and AV 500 spectrometers. The ¹H and ¹³C NMR chemical shifts are referenced to TMS (δ TMS = 0 (¹H)), and to the solvent resonance (δCDCl₃ = 77.0 (¹³C)). EI mass spectra were recorded on an AMD 402 spectrometer (70 eV, AMD Intectra GmbH). IR spectra were recorded on a FT-IR Nicolet 6700 (Thermo ELECTRON CORPORATION). The synthesis of compounds **2a-b** and **7a-d** have been already described in earlier publications (K. Alex, N. Schwarz, V. Khedkar, I. A. Sayyed, A. Tillack, D. Michalik, J. Holenz, J. L. Diaz, M. Beller, Org. Biomol. Chem. 2008 , 6, 1802-1807; and N. Schwarz, A. Tillack, K. Alex, I. A. Sayyed, R. Jackstell, M. Beller, Tetrahedron Lett. 2007, 48, 2897-2900)

### Three step synthesis of the starting material 5-bromo-3-(2-diethylamino-ethoxy)-2-methyl-indole-1-carboxylic acid tert-butyl ester (2c):

### 5-Bromo-3-(tert-butyl-dimethyl-silanyloxy)-2-methyl-1H-indole (1a)

*Step 1:* In a round bottomed flask under an argon atmosphere (4-bromophenyl)hydrazine (0.09 mol), *tert*-butyldimethylsilyloxy-2-propyne (0.07 mol) and ZnCl₂ (0.09 mmol) were dissolved in 50 mL THF. The reaction mixture was heated to 100 °C under reflux for 24 h. After removal of the solvent the mixture was cleaned by column chromatography (eluent: ethyl acetate gradient 0-25% in heptane with 1-2% triethylamine). The isolated product gave a light brown solid in a yield of 50%

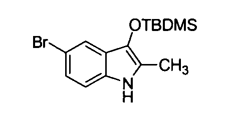

¹H NMR (300.13 MHz, CDCl₃): δ = 7.53 (d, 1H, *J* = 1.9 Hz); 7.37 (br, 1H, NH); 7.14 (dd, 1H, *J* = 8.5 Hz, *J* = 1.9 Hz); 7.04 (d, 1H, *J* = 8.5 Hz); 2.30 (s, 3H); 1.07 (s, 9H); 0.15 (s, 6H) ppm. ¹³C NMR (75.5 MHz, CDCl₃): δ = 131.3 (Cq); 130.4 (Cq); 124.5 (Cq); 123.8; 121.6 (Cq); 119.7; 112.1 (Cq); 111.8; 25.7; 18.1 (Cq); 10.6; -4.2 ppm. MS (EI, 70 eV) m/z (rel. intensity): 340 (22), 339 (M⁺, 93), 285 (17), 284 (10), 283 (16), 205 (5), 204 (19), 203 (100), 188 (19), 145 (9), 144 (47), 73 (41). HRMS Calcd. for C₁₅H₂₂BrNOSi: 339.06485. Found: 339.064659. FTIR (KBr): 3399, 2954, 2929, 2856, 1724, 1471, 1314, 1285, 1255, 1238, 1156, 888, 861, 838, 791, 780, 583 cm⁻¹.

### 5-Bromo-3-(tert-butyl-dimethyl-silanyloxy)-2-methyl-indole-1-carboxylic acid tert-butyl ester (1b)

*Step 2:* In a round bottomed flask under an argon atmosphere 5-bromo-3-(*tert*-butyldimethyl-silanyloxy)-2-methyl-1*H*-indole **1a** (10 mmol), di-*tert*-butyl-dicarbonate (12 mmol) and DMAP (0.35 mmol) were dissolved in dry THF and stirred over night at room temperature. After complete conversion (TLC control) the solvent was removed and the residue was cleaned by column chromatography (eluent: ethyl acetate gradient 0-25% in heptane). The isolated product gave a light yellow solid material in a yield of 90%.

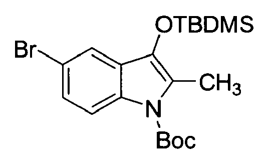

¹H NMR (300.13 MHz, CDCl₃): δ = 7.99 (d, 1H, *J* = 8.9 Hz); 7.49 (d, 1H, *J* = 2.0 Hz); 7.29 (dd, 1H, *J* = 8.9 Hz, *J* = 2.0 Hz); 2.44 (s, 3H); 1.66 (s, 9H); 1.08 (s, 9H); 0.17 (s, 6H) ppm. ¹³C NMR (75.5 MHz, CDCl₃): δ = 150.5 (Cq); 134.7 (Cq); 132.2; 127.0 (Cq); 126.3; 123.6; 119.6 (Cq); 116.9 (Cq); 115.6 (Cq); 83.7 (Cq); 28.3; 25.8; 18.2 (Cq); 12.9; -4.1 ppm. MS (EI, 70 eV) m/z (rel. intensity): 441 (11), 440 (3), 439 (M⁺; 11), 386 (24), 385 (100), 384 (23), 383 (97), 341 (58), 340 (13), 339 (57), 284 (12), 283 (14), 282 (12), 204 (12), 203 (64), 145 (4), 144 (26), 75 (15), 74 (6), 73 (83), 59 (12), 58 (7), 57 (91). HRMS Calcd. for C₂₀H₃₀BrNO₃Si: 439.11728. Found: 439.116436. FTIR (ATR): 3077, 3004, 2976, 2958, 2930, 2896, 2858, 1724, 1454, 1360, 1320, 1267, 1251, 1217, 1150, 1129, 1068, 1010, 887, 835, 820, 799, 783, 764, 729 cm⁻¹.

### 5-Bromo-3-(2-diethylamino-ethoxy)-2-methyl-indole-1-carboxylic acid tert-butyl ester (2c)

*Step 3:* In a round bottomed flask 5-bromo-3-(*tert*-butyl-dimethyl-silanyloxy)-2-methyl-indole-1-carboxylic acid *tert*-butyl ester **1b** (3.4 mmol) and tetrabutylammoniumfluoride (TBAF) (4.1 mmol) were dissolved in dry THF and stirred for 10 min at room temperature. After Na₂CO₃ (6.8 mmol) was added, the reaction mixture was stirred for further 10 min at room temperature. Finally (2-chloro-ethyl)-diethyl-amine (6.8 mmol) was added and the solution was heated up to 45 °C for 4 h. After removal of the solvent the residue was cleaned by column chromatography (eluent CH₂Cl₂/ethanol 20:1). The isolated product obtained as pale yellow oil with a yield of 68 %.

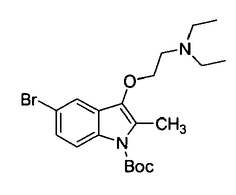

¹H NMR (300.13 MHz, CDCl₃): δ = 8.00 (d, 1H, *J* = 8.9 Hz); 7.64 (d, 1H, *J* = 2.0 Hz); 7.29 (dd, 1H, *J* = 8.9 Hz, *J* = 2.0 Hz); 4.05 (t, 2H, *J* = 6.3 Hz); 2.84 (t, 2H, *J* = 6.3 Hz); 2.63 (q, 4H, *J* = 7.2 Hz); 2.49 (s, 3H); 1.65 (s, 9H); 1.06 (t, *J* = 7.2 Hz, 6H) ppm. ¹³C NMR (75.5 MHz, CDCl₃): δ = 150.4 (Cq); 138.9 (Cq); 132.4 (Cq); 126.6 (Cq); 126.4; 126.0 (Cq); 119.5; 117.1; 115.8 (Cq); 84.0 (Cq); 72.7 (CH₂); 52.8 (CH₂); 47.5 (CH₂); 28.2; 12.5; 11.7 ppm. MS (EI, 70 eV) m/z (rel. intensity):426 (1), 425 (M⁺,1), 424 (1), 385 (7), 384 (1), 383 (6), 341 (3), 279 (4), 225 (6), 197(4), 167 (11), 149 (31), 101 (25), 100 (100), 72 (17), 57 (76). HRMS Calcd. for C₂₀H₂₉BrN₂O: 425.14362. Found: 425.14343. FTIR (ATR): 3052, 2969, 2930, 2873, 2805, 1728, 1453, 1369, 1348, 1319, 1221, 1172, 1148, 1130, 1116, 1061, 1015, 765, 745 cm⁻¹.

### [2-(5-Bromo-2-methyl-1H-indol-3-yloxy)-ethyl]-diethyl-amine (2d)

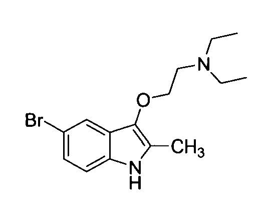

¹H NMR (300.13 MHz, CDCl₃): δ = 7.67 (d, 1H, *J* = 2.0 Hz); 7.60 (br, 1H, NH); 7.16 (dd, 1H, *J* = 8.5 Hz, *J* = 2.0 Hz); 7.06 (d, 1H, *J* = 8.5 Hz); 4.09 (t, 2H, *J* = 6.3 Hz); 2.86 (t, 2H, *J* = 6.3 Hz); 2.65 (q, 4H, *J* = 7.2 Hz); 2.35 (s, 3H); 1.07 (t, 6H, *J* = 7.2 Hz) ppm. ¹³C NMR (75.5 MHz, CDCl₃): δ = 134.7 (Cq); 131.3 (Cq); 124.1 (Cq); 123.9; 123.7 (Cq); 119.6; 112.4 (Cq); 112.1; 72.7 (CH₂); 52.7 (CH₂); 47.4 (CH₂); 11.7; 10.3 ppm. MS (EI, 70 eV) m/z (rel. intensity): 324 (M⁺, 0.3), 226 (3), 225 (1), 224 (3), 145 (3), 117 (5), 101 (8), 100 (100), 87 (3), 86 (47). HRMS Calcd. for C₁₅H₂₁BrN₂OSi: 324.08318. Found: 324.081805. FTIR (ATR): 3410, 3300, 2964, 2919, 2850, 1453, 1284, 1234, 1154, 1122, 1034, 862, 791, 732, 668 cm⁻¹.

### Compounds 3a-f; General procedure for the coupling synthesis of 5-amino-3-(N,N-diethylamino-ethoxy)indole derivatives 2a-c:

In an Ace-pressure tube under an argon atmosphere 5-bromo-3-(*N,N*-diethylamino-ethoxy) indole derivative **2a-c** (1 mmol), amine (1.3 mmol), Pd(OAc)₂ (2 mol%; 6 mol% for **3c** and **3e**), *N*-phenyl-2-(diadamantylphosphino)pyrrole L (4 mol%; 12 mol% for **3c** and **3e**) and lithium hexamethyldisilazane (LiHMDS) (1.3 mmol or1.5 mmol Cs₂CO₃ for **3c** and **3e**) were dissolved in toluene (3 mL). The pressure tube was fitted with a Teflon cap and heated up to 100 °C for 20 h. After removal of the solvent in vacuo, the corresponding indole product was isolated as oil by column chromatography (eluent: ethyl acetate gradient 0-20% in heptane with 1-2% triethylamine).

### Benzyl-[3-(2-diethylamino-ethoxy)-1,2-dimethyl-1H-indol-5-yl]-amine (3a)

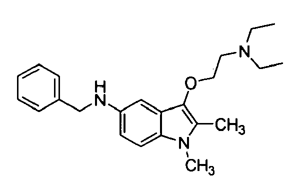

¹H NMR (300.13 MHz, CDCl₃): δ = 7.44-7.26 (m, 5H); 7.05 (d, 1H, *J* = 8.4 Hz); 6.77 (d, 1H, *J* = 2.4 Hz); 6.59 (dd, 1H, *J* = 8.4 Hz, *J* = 2.4 Hz); 4.37 (s, 2H); 4.08 (t, 2H, *J* = 6.5 Hz); 3.54 (s, 3H); 3.30 (br, 1H, NH); 2.90 (t, 2H, *J* = 6.5 Hz); 2.69 (q, 4H, *J* = 7.2 Hz); 2.31 (s, 3H); 1.09 (t, 6H, *J* = 7.2 Hz) ppm. ¹³C NMR (75.5 MHz, CDCl₃): δ = 141.6 (Cq); 140.1 (Cq); 134.2 (Cq); 128.6 (Cq); 128.5; 127.7; 127.0; 125.3 (Cq); 121.4 (Cq); 110.5; 109.4; 99.0; 72.0 (CH₂); 52.5 (CH₂); 49.8 (CH₂); 47.5 (CH₂); 29.4; 11.5; 8.9 ppm. MS (EI, 70 eV) m/z (rel. intensity): 366 (2), 365 (8), 365 (M⁺, 1), 288 (1), 262 (19), 261 (6), 250 (3), 249 (2), 175 (4), 174 (9), 173 (26), 118 (1), 101 (7), 100 (100), 99 (2), 86 (9), 44 (7). HRMS Calcd. for C₂₃H₃₁N₃O: 365.24616. Found: 365.245156. FTIR (ATR): 3392, 3060, 3027, 2966, 2630, 2871, 2820, 1774, 1451, 1370, 1256, 1169, 1069, 1028, 787, 737, 670 cm⁻¹.

### Benzyl-[1-benzyl-3-(2-diethylamino-ethoxy)-2-methyl-1H-indol-5-yl]-amine (3b)

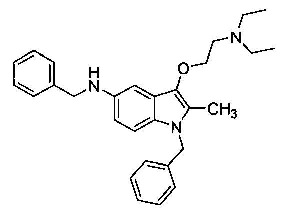

¹H NMR (300.13 MHz, CDCl₃): δ = 7.44-7.19 (m, 8H); 6.97 (d, 1H, *J* = 8.7 Hz); 6.92 (dd, 2H, *J* = 7.7 Hz, *J* = 1.7 Hz); 6.82 (d, 1H, *J* = 2.1 Hz); 6.53 (dd, 1H, *J* = 8.7 Hz, *J* = 2.1 Hz); 5.17 (s, 2H); 4.35 (s, 2H); 4.09 (t, 2H, *J* = 6.6 Hz); 2.86 (t, 2H, *J* = 6.6 Hz); 2.63 (q, 4H, *J* = 7.1 Hz); 2.24 (s, 3H); 1.05 (t, 6H, *J* = 7.1 Hz) ppm. ¹³C NMR (75.5 MHz, CDCl₃): δ = 141.8 (Cq); 140.0 (Cq); 138.4 (Cq); 134.8 (Cq); 128.7; 128.5; 128.4 (Cq); 127.7; 127.1; 125.9; 125.1 (Cq); 121.9 (Cq); 110.7; 110.0; 99.0; 72.5 (CH₂); 52.7 (CH₂); 49.7 (CH₂); 47.6 (CH₂); 46.4 (CH₂); 11.8; 8.9 ppm. MS (EI, 70 eV) m/z (rel. intensity): 441 (M⁺, 1), 252 (3), 251 (4), 181 (2), 161 (3), 145 (2), 100 (100), 91 (54). HRMS Calcd. for C₂₉H₃₅N₃O: 441.27746. Found: 441.277177. FTIR (KBr): 3061, 3028, 2968, 2930, 2871, 2813, 1625, 1494, 1452, 1373, 1354, 1269, 1168, 1028, 733, 697 cm⁻¹.

### 5-Benzylamino-3-(2-diethylamino-ethoxy)-2-methyl-indole-1-carboxylic acid tert-butyl ester (3c)

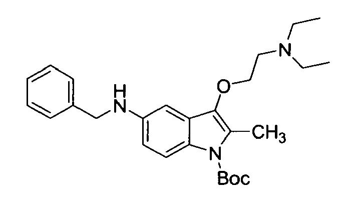

¹H NMR (300.13 MHz, CDCl₃): δ = 7.91 (d, 1H, *J* = 8.9 Hz); 7.41-7.25 (m, 5H); 6.70 (d, 1H, *J* = 2.4 Hz); 6.60 (dd, 1 H, *J* = 8.9 Hz, *J* = 2.4 Hz); 4.37 (s, 2H); 4.01 (t, 2H, *J* = 6.5 Hz); 2.82 (t, 2H, *J* = 6.5 Hz); 2.61 (q, 4H, *J* = 7.1 Hz); 2.47 (s, 3H); 1.64 (s, 9H); 1.04 (t, 6H, *J* = 7.1 Hz) ppm. ¹³C NMR (75.5 MHz, CDCl₃): δ =150.8 (Cq); 144.0 (Cq); 139.6 (Cq); 139.6 (Cq); 128.5; 127.5; 127.1 (Cq); 125.5 (Cq); 125.2 (Cq); 116.4; 111.5; 99.1; 82.9 (Cq); 72.1 (CH₂); 52.7 (CH₂); 49.0 (CH₂); 47.5 (CH₂); 28.3; 12.4; 11.8 ppm MS (EI, 70 eV) m/z (rel. intensity): 451 (M⁺, 1), 351 (3), 252 (18), 251 (5), 250 (6), 161 (22), 160 (4), 159 (5), 134 (3), 133 (9), 101 (11), 100 (100), 92 (5), 91 (27), 86 (40), 85 (5), 72 (18), 71 (10), 57 (16), 56 (23), 44 (51), 43 (9), 42 (11), 40 (32). HRMS Calcd. for C₂₇H₃₇N₃O₃: 451.28294. Found: 451.283100. FTIR (ATR): 3416, 3062, 3028, 2969, 2927, 2872, 2810, 1720, 1470, 1452, 1367, 1330, 1275, 1221, 1168, 1129, 1062, 734, 697 cm⁻¹.

### [3-(2-Diethylamino-ethoxy)-1,2-dimethyl-1H-indol-5-yl]-hexyl-amine (3d)

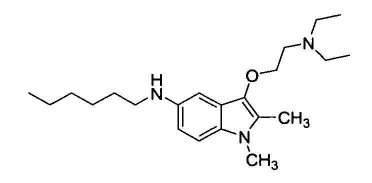

¹H NMR (300.13 MHz, CDCl₃): δ = 7.03 (d, 1 H, *J* = 8.6 Hz); 6.73 (d, 1 H, *J* = 2.1 Hz); 6.56 (dd, 1 H, *J* = 8.6 Hz, *J* = 2.1 Hz); 4.17 (t, 2H, *J* = 6.2 Hz); 3.53 (s, 3H); 3.14 (t, 2H, *J* = 7.1 Hz); 3.01 (t, 2H, *J* = 6.2 Hz); 2.82 (q, 4H, *J* = 7.1 Hz); 2.31 (s, 3H); 1.71-1.58 (m, 2H); 1.50-1.26 (m, 6H); 1.17 (t, 6H, *J* = 7.1 Hz); 0.90 (t, 3H, *J* = 6.7 Hz) ppm. ¹³C NMR (75.5 MHz, CDCl₃): δ = 142.0 (Cq), 134.0 (Cq); 128.5 (Cq); 125.1 (Cq); 121.3 (Cq); 110.7; 109.4; 98.8; 71.6 (CH₂); 52.4 (CH₂); 47.6 (CH₂); 45.6 (CH₂); 31.7 (CH₂); 29.7 (CH₂); 29.3; 27.0 (CH₂); 22.6 (CH₂); 14.0; 11.0; 9.0 ppm. MS (EI, 70 eV) m/z (rel. intensity): 359 (M⁺, 5), 259 (6), 244 (1), 187 (3), 173 (4), 100 (100), 86 (6), 44 (5). HRMS Calcd. for C₂₂H₃₇N₃O: 359.29311. Found: 359.292382. FTIR (ATR): 3313, 2959, 2932, 2856, 2808, 2590, 2461, 1626, 1475, 1460, 1367, 1253, 1176, 1024, 781, 732 cm⁻¹.

### 3-(2-Diethylamino-ethoxy)-5-hexylamino-2-methyl-indole-1-carboxylic acid tert-butyl ester (3e)

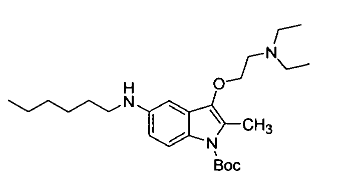

¹H NMR (400.13 MHz, CDCl₃): δ = 7.89 (d, 1H, *J* = 8.8 Hz); 6.66 (d, 1H, *J* = 2.3 Hz); 6.55 (dd, 1H, *J* = 8.8 Hz, *J* = 2.3 Hz); 4.06 (t, 2H, *J* = 6.4 Hz); 3.13 (t, 2H, *J* = 7.2 Hz); 2.86 (t, 2H, *J* = 6.4 Hz); 2.64 (q, 4H, *J* = 7.2 Hz); 2.46 (s, 3H); 1.63 (s, 9H); 1.50-1.25 (m, 8H); 1.06 (t, 6H, *J* = 7.2 Hz); 0.89 (t, 3H, *J* = 6.7 Hz) ppm. ¹³C NMR (100.6 MHz, CDCl₃): δ = 150.9 (Cq); 144.5 (Cq); 139.7 (Cq); 127.0 (Cq); 125.4 (Cq); 125.3 (Cq); 116.4; 111.6; 98.9; 82.9 (Cq); 72.2 (CH₂); 52.8 (CH₂); 47.6 (CH₂); 44.9 (CH₂); 31.7 (CH₂); 29.7 (CH₂); 28.4; 26.9 (CH₂); 22.7 (CH₂); 14.1; 12.4; 11.8 ppm. MS (EI, 70 eV) m/z (rel. intensity): 446 (1), 445 (M⁺, 5), 346 (2), 345 (9), 290 (3), 246 (16), 245 (7), 244 (3), 176 (3), 175 (23), 174 (5), 147 (4), 146 (3), 145 (7), 100 (100), 99 (8), 98 (8), 97 (17), 86 (42), 85 (10), 84 (12), 83 (21), 58 (8), 57 (45), 44 (58). HRMS Calcd. for C₂₆H₄₃N₃O₃: 445.32989. Found: 445.330462. FTIR (ATR) 3400, 2964, 2927, 2856, 1721, 1367, 1329, 1314, 1221, 1168, 1127, 1061, 1018, 846, 764, 678 cm⁻¹_{.}

### [3-(2-Diethylamino-ethoxy)-1,2-dimethyl-1H-indol-5-yl]-[2-(4-fluoro-phenyl)-ethyl]-amine (3f)

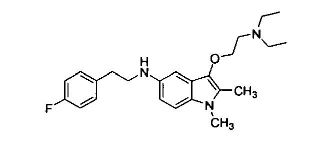

¹H NMR (500.13 MHz, CDCl₃): δ = 7.19 (m, 2H); 7.05 (d, 1H, *J* = 8.6 Hz); 7.00 (m, 2H); 6.78 (d, 1H, *J* = 2.0 Hz); 6.51 (dd, 1 H, *J* = 8.6 Hz, *J* = 2.0 Hz); 4.13 (t, 2H, *J* = 6.3 Hz); 4.01 (br, 1H, NH); 3.54 (s, 3H); 3.43 (t, 2H, *J* = 6.9 Hz); 2.95-2.91 (m, 4H); 2.72 (q, 4H, *J* = 7.1 Hz); 2.32 (s, 3H); 1.10 (t, 6H, *J* = 7.1 Hz) ppm. ¹³C NMR (125.8 MHz, CDCl₃): δ = 161.5 (d, *J* = 244 Hz, Cq); 141.1 (Cq); 135.3 (d, *J* = 3.0 Hz, Cq); 134.2 (Cq); 130.2 (d, *J* = 7.5 Hz); 128.7 (Cq); 125.2 (Cq); 121.5 (Cq); 115.3 (d, *J* = 21.0 Hz); 110.8; 109.4; 99.4; 72.3; 52.7; 47.5; 46.7; 34.8; 29.3; 11.5; 8.9 ppm. MS (EI, 70 eV) m/z (rel. intensity): 397 (M⁺, 4), 297 (3), 281 (1), 188 (6), 173 (7), 159 (4), 130 (1), 100 (100), 86 (8), 44 (7). HRMS Calcd. for C₂₄H₃₂FN₃O: 397.25239. Found: 397.251869. FTIR (ATR): 2966, 2930, 2871, 2821, 1508, 1476, 1446, 1371, 1256, 1220, 1169, 1157, 1067, 1014, 824, 786 cm⁻¹.

### Compounds 4a-c; General procedure for the hydrogenation of 5-benzylamino-3-(N,N-diethylamino-ethoxy)indole derivatives 3a-c:

In an 20 mL autoclave 5-benzylamino-3-(*N,N*-diethylamino-ethoxy)indole derivative(1 mmol) was dissolved in 40 mL ethanol, before Pd/C (10%) (200 mg) was added. Under a pressure of 50 bar (5 bar for **4c**) H₂, the reaction mixture was stirred for 8 h (3.5 h for **4c**) at room temperature (60 °C for **4c**). After removal of the solvent in vacuo, the hydrogenated indole derivative was isolated as an oil by column chromatography (eluent heptane/ethylacetate (5:1) with 5% triethylamine).

### 3-(2-Diethylamino-ethoxy)-1,2-dimethyl-1H-indol-5-ylamine (4a)

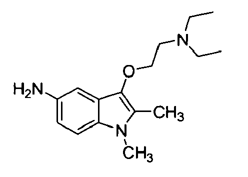

¹H NMR (300.13 MHz, COCl₃): δ = 6.95 (d, 1H, *J* = 8.6 Hz); 6.78 (d, 1H, *J* = 2.1 Hz); 6.51 (dd, 1H, *J* = 8.6 Hz, *J* = 2.1 Hz); 3.99 (t, 2H, *J* = 6.5 Hz); 3.45 (s, 3H); 3.21 (br, 2H, NH₂); 2.79 (t, 2H, *J* = 6.5 Hz); 2.56 (q, 4H, *J* = 7.1 Hz); 2.23 (s, 3H); 0.99 (t, 6H, *J* = 7.1 Hz) ppm. ¹³C NMR (75.5 MHz, CDCl₃): δ = 138.6 (Cq); 134.0 (Cq); 129.1 (Cq); 125.4 (Cq); 121.5 (Cq); 111.4; 109.2; 102.2; 72.6 (CH₂); 52.7 (CH₂); 47.5 (CH₂); 29.3; 11.8; 8.8 ppm. MS (EI, 70 eV) m/z (rel. intensity): 275 (M⁺, 4), 176 (3), 175 (12), 160 (4), 159 (2), 101 (7), 100 (100), 91 (4), 86 (13), 72 (8), 44 (9). HRMS Calcd. for C₁₆H₂₅N₃O: 275.19921. Found: 275.199332. FTIR (KBr): 3420, 3342, 3219, 2966, 2933, 2872, 2819, 1628, 1495, 1470, 1372, 1322, 1257, 1165, 1068, 793 cm⁻¹_{.}

### 1-Benzyl-3-(2-diethylamino-ethoxy)-2-methyl-1H-indol-5-ylamine (4b)

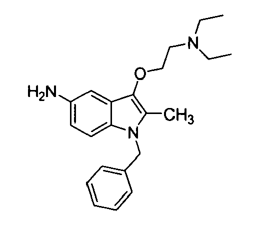

¹H NMR (300.13 MHz, CDCl₃): δ = 7.23-7.20 (m, 3H); 6.97 (d, 1 H, *J* = 8.6 Hz); 6.93-6.90 (m, 3H); 6.53 (dd, 1 H, *J* = 8.6 Hz, *J* = 2.2 Hz); 5.18 (s, 2H); 4.11 (t, 2H, *J* = 6.6 Hz); 3.10 (br, 2H, NH₂); 2.88 (t, 2H, *J* = 6.6 Hz); 2.65 (q, 4H, *J* = 7.1 Hz); 2.25 (s, 3H); 1.07 (t, 6H, *J* = 7.1 Hz) ppm. ¹³C NMR (75.5 MHz, CDCl₃): δ = 138.9 (Cq); 138.3 (Cq); 134.5 (Cq); 129.0 (Cq); 128.7; 127.1; 125.9; 125.4 (Cq); 121.9 (Cq); 111.7; 109.8; 102.3; 72.6 (CH₂); 52.7 (CH₂); 47.5 (CH₂); 46.4 (CH₂); 11.8; 8.9 ppm. MS (EI, 70 eV) m/z (rel. intensity): 351 (M⁺, 3), 251 (2), 236 (1), 159 (1), 132 (2), 100 (100), 91 (26). HRMS Calcd. for C₂₂H₂₉N₃O: 351.23051. Found: 351.230802. FTIR (KBr): 3035, 2970, 2921, 1626, 1490, 1456, 1437, 1371, 1356, 1329, 1268, 1165, 732 cm⁻¹_{.}

### 5-Amino-3-(2-diethylamino-ethoxy)-2-methyl-indole-1-carboxylic acid tert-butyl ester (4c)

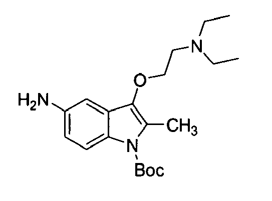

¹H NMR (300.13 MHz, CDCl₃): δ = 7.91 (d, 1H, *J* = 8.9 Hz); 6.79 (d, 1H, *J* = 2.2 Hz); 6.62 (dd, 1H, *J* = 8.9 Hz, *J* = 2.2 Hz); 4.06 (t, 2H, *J* = 6.4 Hz); 3.59 (br, 2H, NH₂); 2.86 (t, 2H, *J* = 6.4 Hz); 2.64 (q, 4H, *J* = 7.1 Hz); 2.47 (s, 3H); 1.65 (s, 9H); 1.07 (t, 6H, *J* = 7.1 Hz) ppm. ¹³C NMR (75.5 MHz, CDCl₃): δ = 150.8 (Cq); 141.7 (Cq); 139.4 (Cq); 127.8 (Cq); 125.7 (Cq); 125.3 (Cq); 116.4; 112.8; 102.1; 83.1 (Cq); 72.2 (CH₂); 52.8 (CH₂); 47.5 (CH₂); 28.5; 12.4; 11.8 ppm. MS (EI, 70 eV) m/z (rel. intensity): 362 (2), 361 (M⁺, 8), 261 (3), 162 (9), 161 (8), 147 (2), 146, (5), 145 (5), 101 (19), 100 (100), 86 (67), 72 (17), 57 (40). HRMS Calcd. for C₂₀H₃₁N₃O₃: 361.23599. Found: 361.235080. FTIR (ATR): 3443, 3369, 3211, 3027, 2969, 2929, 2873, 2810, 1720, 1365, 1327, 1254, 1218, 1166, 1129, 1060, 1033 cm⁻¹.

### Compounds 5a-h: General procedure for the sulfonylation of 5-amino-3-(N,N-diethylamino-ethoxy)indole derivatives 3c, 3e, 4a-c:

In an Ace-pressure tube under an argon atmosphere the corresponding 5-amino-substituted-3-(*N,N*-diethylamino-ethoxy)-indole derivative (1 mmol) and the arylsulfonylchloride (1 mmol) (see Table 3) were dissolved in 5 ml triethylamine (for the synthesis of **5a-b, 5e-f**) and heated at 40°C for 2 h. In the case of **5c** and **5g** 10 mL CH₂Cl₂ as solvent and 2 equiv CS₂CO₃ as base at rt were used. The pressure tube was fitted with a Teflon cap. After removal of the solvent in vacuo the corresponding indole product was isolated by column chromatography as oil or solid material with heptane/ethyl acetate (5:1) and 1% triethylamine.

### 2,1,3-Benzothiadiazole-5-sulfonic acid [3-(2-diethylamino-ethoxy)-1,2-dimethyl-1H-indol-5-yl]-amide (5a)

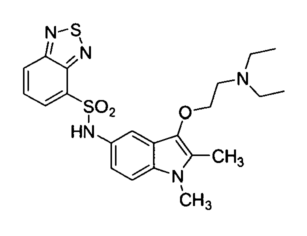

¹H NMR (300.13 MHz, CDCl₃): δ = 8.11 (dd, 1H, *J* = 8.9 Hz, *J* = 1.0 Hz); 8.08 (dd, 1H, *J* = 7.0 Hz, *J* = 1.0 Hz); 7.53 (dd, 1H, *J* = 8.9 Hz, *J* = 7.0 Hz); 7.11 (d, 1 H, *J* = 2.0 Hz); 6.93 (d, 1 H, *J* = 8.8 Hz); 6.69 (dd, 1 H, *J* = 8.8 Hz, *J* = 2.0 Hz); 3.97 (t, 2H, *J* = 6.1 Hz); 3.47 (s, 3H); 2.89 (t, 2H, *J* = 6.1 Hz); 2.76 (q, 4H, *J* = 7.2 Hz); 2.25 (s, 3H); 1.13 (t, 6H, *J* = 7.2 Hz) ppm. ¹³C NMR (75.5 MHz, CDCl₃): δ = 155.1 (Cq); 149.2 (Cq); 134.4 (Cq); 133.0 (Cq); 132.0; 130.8 (Cq); 123.2; 127.0 (Cq); 126.4 (Cq); 126.2; 120.6 (Cq); 116.9; 111.7; 109.1; 72.0 (CH₂); 52.4 (CH₂); 47.5 (CH₂); 29.3; 11.1; 8.8 ppm. MS (EI, 70 eV) m/z (rel. intensity): 474 (1), 473 (M⁺, 2), 472 (1), 374 (6), 373 (9), 372 (2), 176 (12), 175 (52), 174 (38), 173 (22), 161 (4), 160 (11), 159 (11), 148 (5), 147 (11), 146 (3), 145 (5), 137 (3), 136 (12), 135 (2), 101 (37), 100 (100), 99 (5), 98 (7), 86 (72), 85 (3), 84 (6), 73 (5), 72 (30), 71 (8), 70 (7), 69 (6), 57 (9), 56 (15), 55 (5). HRMS Calcd. for C₂₂H₂₇N₅O₃S₂: 473.15498. Found: 473.154213. FTIR (KBr): 3448, 3203, 3085, 2962, 2925, 2835, 1521, 1488, 1374, 1347, 1332, 1271, 1252, 1213, 1162, 1143, 966, 834, 819, 764, 733, 669, 612, 594, 482 cm⁻¹_{.}

### 2,1,3-Benzothiadiazole-4-sulfonic acid [1-benzyl-3-(2-diethyl-aminoethoxy)-2-methyl-1H-indol-5-yl]-amide (5b)

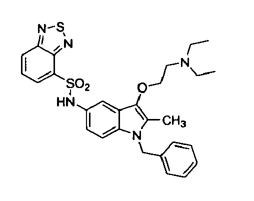

¹H NMR (300.13 MHz, CDCl₃): δ = 8.14 (dd, 1H, *J* = 8.8 Hz, *J* = 1.1 Hz); 8.10 (dd, 1H, *J* = 7.0 Hz, *J*= 1.2 Hz); 7.56 (dd, 1 H, *J* = 8.8 Hz, *J* = 7.0 Hz); 7.24-7.17 (m, 3H); 7.13 (d, 1 H, *J* = 2.0 Hz); 6.90 (d, 1H, *J* = 8.7 Hz); 6.86-6.80 (m, 2H); 6.66 (dd, 1H, *J* = 8.7 Hz, *J* = 2.0 Hz); 5.11 (s, 2H); 3.93 (t, 2H, *J* = 6.4 Hz); 2.77 (t, 2H, *J* = 6.4 Hz); 2.61 (q, 4H, *J* = 7.1 Hz); 2.20 (s, 3H); 1.05 (t, 6H, *J* = 7.1 Hz) ppm. ¹³C NMR (75.5 MHz, CDCl₃): δ = 155.2 (Cq); 149.3 (Cq); 137.4 (Cq); 135.1 (Cq); 132.1 (Cq); 132.0; 131.0 (Cq); 128.7; 128.3; 127.4; 127.2 (Cq); 126.3 (Cq); 126.3; 125.8; 121.2 (Cq); 117.4; 112.1; 109.6; 72.8 (CH₂); 52.6 (CH₂); 47.5 (CH₂); 46.5 (CH₂); 11.8; 9.0 ppm. MS (EI, 70 eV) m/z (rel. intensity): 550 (1), 549 (M⁺,1), 351 (1), 252 (4), 251 (7), 250 (2), 236 (1), 235 (2), 162 (1), 161 (3), 160 (2), 137 (1), 136 (7), 135 (1), 101 (9), 100 (100), 99 (2), 92 (4), 91 (44), 86 (15), 85 (2), 58 (3), 57 (5), 56 (5), 44 (12). HRMS Calcd. for C₂₈H₃₁N₅O₃S₂: 549.18628. Found: 549.187203. FTIR (KBr): 3452, 2970, 2929, 2872, 1626, 1495, 1474, 1454, 1375, 1353, 1289, 1210, 1155, 1142, 967, 754, 731, 608 cm⁻¹.

### 5-[(2,1,3-Benzothiadiazole-4-sulfonyl)-hexyl-amino]-3-(2-dieth-ylamino-ethoxy)-2-methyl-indole-1-carboxylic acid tert-butyl ester (5c)

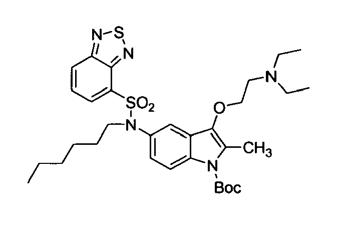

¹H NMR (400.13 MHz, CDCl₃): δ = 8.15 (dd, 1H, *J* = 8.8 Hz, *J* = 1.3 Hz); 7.94 (d, 1H. *J* = 9.0 Hz); 7.91 (dd, 1H, *J* = 7.1 Hz, *J* = 1.3 Hz); 7.51 (dd, 1H, *J* = 8.8 Hz , *J* = 7.1 Hz); 7.06 (d, 1H, *J* = 2.0 Hz); 6.78 (dd, 1H, *J* = 9.0 Hz, *J* = 2.0 Hz); 4.02 (t, 2H, *J* = 7.1 Hz); 3.83 (t, 2H, *J* = 6.2 Hz); 2.72 (t, 2H, *J* = 6.2 Hz); 2.57 (q, 4H, *J* = 7.2 Hz); 2.45 (s, 3H); 1.62 (s, 9H); 1.50-1.25 (m, 8H); 1.01 (t, 6H, *J* = 7.2 Hz); 0.84 (m, 3H) ppm. ¹³C NMR (100.6 MHz, CDCl₃): δ = 155.5 (Cq); 150.4 (Cq); 149.7 (Cq); 139.4 (Cq); 133.0 (Cq); 132.7 (Cq); 132.6; 131.7 (Cq); 128.1; 126.5 (Cq); 126.0; 124.6 (Cq); 124.5; 117.6; 116.1; 84.0 (Cq); 72.5 (CH₂); 52.9 (CH₂); 52.6 (CH₂); 47.4 (CH₂); 31.4 (CH₂); 28.9 (CH₂); 28.2; 26.1 (CH₂); 22.5 (CH₂); 14.0; 12.4; 11.7 ppm. MS (EI, 70 eV) m/z (rel. intensity): 643 (M⁺, 1), 346 (1), 345 (2), 246 (5), 245 (3), 244 (2), 187 (2), 186 (1), 169 (3), 168 (10), 167 (2), 147 (2), 146 (1), 145 (3), 101 (7), 100 (100), 99 (6), 86 (27), 85 (7), 84 (10), 71 (15), 70 (15), 69 (24), 57 (30), 56 (39), 45 (9), 44 (99), 43 (36). HRMS Calcd. for C₃₂H₄₅N₅O₅S₂: 643.28566. Found: 643.284226. FTIR (neat): 3442, 3093, 3060, 2965, 2929, 2871, 2809, 2725, 1732, 1471, 1353, 1272, 1256, 1225, 1208, 1162, 1070, 1021, 970, 853, 831, 754, 730, 622, 604, 593 cm⁻¹.

### 5-Chloro-3-methyl-benzo[b]thiophene-2-sulfonic acid [3-(2-diethylamino-ethoxy)-1,2-dimethyl-1H-indol-5-yl]-amide (5d)

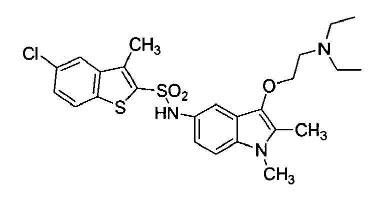

¹H NMR (300.13 MHz, CDCl₃): δ = 7.64 (d, 1H, *J* = 8.6 Hz); 7.60 (d, 1H, *J* = 2.0 Hz); 7.34 (dd, 1H, *J* = 8.6 Hz, *J* = 2.0 Hz); 7.25 (d, 1H, *J* = 2.0 Hz); 7.05 (d, 1H, *J* = 8.7 Hz); 6.91 (dd, 1H, *J* = 8.7 Hz, *J* = 2.0 Hz); 5.56 (br s, 1H, NH); 3.97 (t, 2H, *J* = 6.2 Hz); 3.54 (s, 3H); 2.87 (t, 2H, *J* = 6.2 Hz); 2.74 (q, 4H, *J* = 7.2 Hz); 2.30 (s, 3H); 2.21 (s, 3H); 1.09 (t, 6H, *J* = 7.2 Hz) ppm. ¹³C NMR (75.5 MHz, CDCl₃): δ = 140.5 (Cq); 137.7 (Cq); 136.9 (Cq); 136.1 (Cq); 134.5 (Cq); 132.5 (Cq); 131.1 (Cq); 127.5; 126.7 (Cq); 126.4 (Cq); 123.6; 123.2; 120.6 (Cq); 118.6; 113.5; 109.1; 71.8 (CH₂); 52.4 (CH₂); 47.4 (CH₂); 29.5; 12.1; 10.9; 8.9 ppm. MS (EI, 70 eV) m/z (rel. intensity): 520 (1), 519 (M⁺, 2), 421 (5), 420 (9), 419 (8), 275 (1), 274 (1), 184 (7), 183 (10), 182 (20), 181 (24), 176 (15), 175 (69), 174 (51), 173 (27), 101 (49), 100 (100), 99 (6), 98 (8), 87 (5), 86 (97), 85 (2), 73 (7), 72 (44), 71 (7), 70 (7), 57 (4), 56 (16), 55 (1). HRMS Calcd. for C₂₅H₃₀ClN₃O₃S₂: 519.14116. Found: 519.139652. FTIR (KBr): 3448, 2966, 2921, 2856, 2668, 1857, 1128, 1486, 1373, 1335, 1326, 1277, 1244, 1156, 1117, 1080, 987, 862, 799, 647, 575, 563, 547 cm⁻¹.

### 5-[(6-Chloro-imidazo[2,1-b]thiazole-5-sulfonyl)-hexyl-amino]-3-(2-diethylamino-ethoxy)-2-methyl-indole-1-carboxylic acid tert-butyl ester (5e)

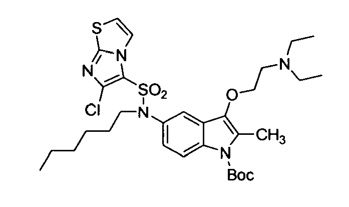

¹H NMR (300.13 MHz, CDCl₃): δ = 8.02 (d, 1H, *J* = 8.9 Hz); 7.19 (d, 1H, *J* = 2.1 Hz); 7.06 (d, 1H, *J* = 4.5 Hz); 6.94 (d, 1H, *J* = 8.9 Hz, *J* = 2.1 Hz); 6.70 (d, 1H, *J* = 4.5 Hz); 3.89 (t, 2H, *J* = 6.1 Hz); 3.76 (t, 2H, *J* = 7.0 Hz); 2.81 (t, 2H, *J* = 6.1 Hz); 2.63 (q, 4H, *J* = 7.1 Hz); 2.47 (s, 3H); 1.64 (s, 9H); 1.46-1.18 (m, 8H); 1.65 (t, 6H, *J* = 7.1 Hz); 0.83 (t, 3H, *J* = 7.0 Hz) ppm. ¹³C NMR (75.5 MHz, CDCl₃): δ = 150.4 (Cq); 149.3 (Cq); 139.4 (Cq); 137.6 (Cq); 133.0 (Cq); 132.5 (Cq); 126.8 (Cq); 124.7 (Cq); 124.1; 120.5; 119.0 (Cq); 117.0; 116.2; 113.4; 84.2 (Cq); 72.5 (CH₂); 52.7 (CH₂); 51.7 (CH₂); 47.4 (CH₂); 31.3 (CH₂); 28.2 (CH₂); 28.2; 26.0 (CH₂); 22.5 (CH₂); 14.0; 12.5; 11.6 ppm. MS (EI, 70 eV) m/z (rel. intensity): 667 (3), 666 (M⁺, 8), 568 (2), 567 (3), 566 (4), 447 (5), 446 (15), 445 (7), 444 (4), 347 (13), 346 (16), 345 (11), 344 (7), 300 (2), 299 (8), 161 (37), 160 (11), 159 (100), 158 (10), 101 (6), 100 (39), 99 (11), 86 (5), 85 (24), 84 (4), 83 (14). HRMS Calcd. for C₃₁H₄₄ClN₅O₅S₂: 666.25452. Found: 666.25510. FTIR (ATR): 3148, 3121, 2959, 2929, 2870, 2858, 1737, 1619, 1453, 1355, 1324, 1269, 1248, 1118, 1133, 1069, 1020, 728, 670 cm⁻¹.

### Benzo[b]thiophene-2-sulfonic acid [3-(2-diethylamino-ethoxy)-1,2-dimethyl-1H-indol-5-yl]-amide (5f)

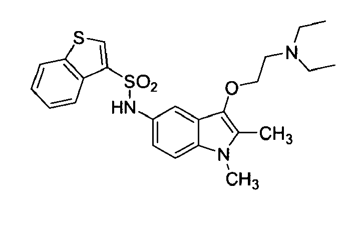

¹H NMR (300.13 MHz, CDCl₃):δ = 8.21-8.14 (m, 1H); 7.93 (s, 1H); 7.87-7.79 (m, 1H); 7.47-7.37 (m, 2H); 7.05 (d, 1H, *J* = 2.0 Hz); 7.00 (d, 1H, *J* = 8.7 Hz); 6.76 (dd, 1H, *J* = 8.7 Hz, *J* = 2.0 Hz); 3.87 (t, 2H, *J* = 6.2 Hz); 3.52 (s, 3H); 2.76 (t, 2H, *J* = 6.2 Hz); 2.60 (q, 4H, *J* = 7.1 Hz); 2.28 (s, 3H); 1.03 (t, 6H, *J* = 7.1 Hz) ppm. ¹³C NMR (75.5 MHz, CDCl₃): δ = 140.1 (Cq); 135.1; 134.7 (Cq); 133.7 (Cq); 132.6 (Cq); 132.5 (Cq); 126.7 (Cq); 126.3 (Cq); 125.6; 125.5; 123.2; 122.8; 120.7 (Cq); 118.8; 113.8; 109.0; 72.5 (CH₂); 52.5 (CH₂); 47.3 (CH₂); 29.4; 11.5; 8.9 ppm. MS (EI, 70 eV) m/z (rel. intensity): 471 (M⁺, 2), 470 (1), 373 (3), 372 (10), 371 (9), 370 (2), 176 (10), 175 (47), 174 (36), 173 (21), 161 (3), 160 (7), 159 (14), 134 (25), 133 (4), 132 (7), 131 (5), 101 (36), 100 (100), 99 (5), 86 (67), 85 (3), 84 (5), 72 (26), 71 (8), 70 (7), 69 (7), 58 (8), 57 (10), 56 (16), 55 (5). HRMS Calcd. for C₂₄H₂₉N₃O₃S₂: 471.16448. Found: 471.164566. FTIR (ATR): 3257, 3104, 2965, 2927, 2872, 2853, 1372, 1276, 1250, 1142, 1064, 076, 797, 756, 731, 706, 668 cm⁻¹.

### 5-[(Benzo[b]thiophene-2-sulfonyl)-benzyl-amino]-3-(2-diethyl-amino-ethoxy)-2-methyl-indole-1-carboxylic acid tert-butyl ester (5g)

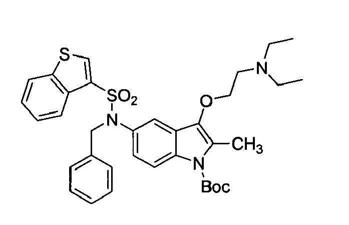

¹H NMR (300.13 MHz, CDCl₃): δ = 7.98 (m, 1H); 7.97 (s, 1H); 7.92 (d, 1H, *J* = 8.8 Hz); 7.88 (m, 1H); 7.46-7.33 (m, 2H); 7.24-7.14 (m, 5H); 6.96 (d, 1H, *J* = 2.1 Hz); 6.85 (dd, 1H, *J* = 8.8 Hz, *J* = 2.1 Hz); 4.89 (s, 2H); 3.70 (t, 2H, *J* = 6.3 Hz); 2.68 (t, 2H, *J* = 6.3 Hz); 2.55 (q, 4H, *J* = 7.1 Hz); 2.44 (s, 3H); 1.62 (s, 9H); 1.02 (t, 6H, *J* = 7.1 Hz) ppm. ¹³C NMR (75.5 MHz, CDCl₃): δ = 150.4 (Cq); 140.1 (Cq); 139.4 (Cq); 136.0 (Cq); 135.0; 134.1 (Cq); 133.0 (Cq); 132.8 (Cq); 132.8 (Cq); 128.7; 128.3; 127.6; 126.2 (Cq); 125.5; 125.5; 125.2; 124.3 (Cq); 124.0; 122.7; 117.6; 115.9; 83.9 (Cq); 72.2 (CH₂); 55.4 (CH₂); 52.6 (CH₂); 47.3 (CH₂); 28.2; 12.4; 11.7 ppm. MS (EI, 70 eV) m/z (rel. intensity): 647 (M⁺, 1), 341 (2), 151 (4), 150 (3), 149 (15), 135 (4), 134 (13), 133 (5), 101 (6), 100 (81), 99 (14), 71 (49), 70 (27), 69 (52), 57 (78), 56 (55), 55 (62), 44 (100), 43 (71). HRMS Calcd. for C₃₅H₄₁N₃O₅S₂: 647.24821. Found: 647.249346. FTIR (neet): 3109, 3064, 3035, 2970, 2925, 2868, 2811, 2255, 1731, 1471, 1455, 1355, 1325, 1259, 1225, 1160, 1139, 1066, 911, 757, 733, 590 cm⁻¹.

### 5-(6-Chloro-imidazo[2,1-b]thiazole-5-sulfonylamino)-3-(2-diethylamino-ethoxy)-2-methyl-indole-1-carboxylic acid tert-butyl ester (5h)

In a round bottomed flask under an argon atmosphere to a solution of 5-amino-3-(2-(diethylamino)ethoxy)-2-methyl-indole-1-carboxylic acid *tert*-butyl ester **4c** (0.5 mmol), NaHCO₃ (2 equiv) and 5-(6-chloro-imidazo[2,1-*b*]thiazole-5-sulfonylchloride (0.5 mmol) were added in 5 mL acetonitrile. The reaction mixture was stirred at room temperature for 15 h. The product was isolated by column chromatography (eluent: ethanol gradient 5-50% in CH₂Cl₂) in a yield of 81% as a white powder.

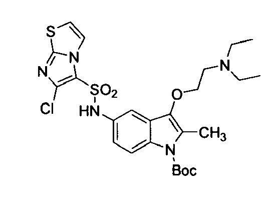

¹H NMR (300.13 MHz, CDCl₃): δ = 7.98 (d, 1H, *J* = 8.8 Hz); 7.70 (d, 1H, *J* = 4.5 Hz); 7.31 (d, 1H, *J* = 2.3 Hz); 7.01 (dd, 1H, *J* = 8.8 Hz, *J* = 2.3 Hz); 6.86 (d, 1H, *J* = 4.5 Hz); 5.24 (br, 1H, NH); 4.01 (t, 2H, *J* = 6.1 Hz); 2.88 (t, 2H, *J* = 6.1 Hz); 2.74 (q, 4H, *J* = 7.1 Hz); 2.45 (s, 3H); 1.63 (s, 9H); 1.09 (t, 6H, *J* = 7.1 Hz) ppm. ¹³C NMR (75.5 MHz, CDCl₃): δ = 150.4 (Cq); 149.5 (Cq); 139.1 (Cq); 137.3 (Cq); 132.0 (Cq); 130.1 (Cq); 126.7 (Cq); 124.6 (Cq); 120.3; 119.8; 118.6 (Cq), 116.4; 114.0; 112.1; 84.0 (Cq); 72.0 (CH₂); 52.5 (CH₂); 47.3 (CH₂); 28.2; 12.4; 11.2 ppm. MS (EI, 70 eV) m/z (rel. intensity): 581 (M⁺; 1), 382 (1), 381 (1), 158 (12), 101 (18), 100 (100), 86 (83), 72 (16), 57 (15), 56 (24). HRMS (ESI⁺, [M+H]⁺) Calcd. for C₂₅H₃₂CIN₅O₅S₅: 582.16061. Found: 582.16052. FTIR (Nujol): 3436, 3109, 2924, 2854, 2716, 1728, 1612, 1542, 1460, 1376, 1323, 1271, 1249, 1179, 1129, 1067, 1022, 933, 725, 622 cm⁻¹.

### Benzo[b]thiophene-3-sulfonic acid benzyl-[3-(2-diethylamino-ethoxy)-2-methyl-1H-indol-5-yl]-amide (6a)

5-[(Benzo[b]thiophene-2-sulfonyl)-benzyl-amino]-3-(2-diethyl-amino-ethoxy)-2-methyl-indole-1-carboxylic acid *tert*-butyl ester **5g** was solved in a 33% methylamine/ethanol solution (10 mL) and stirred at room temperature for 24 h. After removal of the solvent in vacuo the corresponding indole product was isolated by column chromatography (eluent: ethanol gradient 5-20% in CH₂Cl₂) as solid material in a yield of 78%.

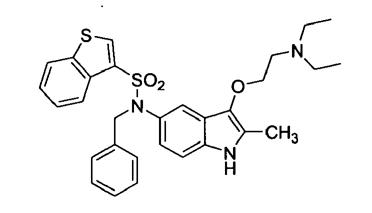

¹H NMR (300.13 MHz, CDCl₃): δ = 7.98 (m, 1H, *J* = 7.3 Hz); 7.97 (s, 1H ); 7.88 (m, 1H, *J* = 7.3 Hz); 7.50 (br s, 1H, NH); 7.44-7.31 (m, 2H); 7.25-7.14 (m, 5H); 6.97 (d, 1H, *J* = 1.9 Hz); 6.94 (d, 1H, *J* = 8.6 Hz); 6.66 (dd, 1H, J = 8.6 Hz, *J* = 1.9 Hz); 4.89 (s, 2H); 3.77 (t, 2H, *J* = 6.2 Hz); 2.71 (t, 2H, *J* = 6.2 Hz); 2.59 (q, 4H, *J* = 7.1 Hz); 2.29 (s, 3H); 1.04 (t, 6H, *J* = 7.1 Hz) ppm. ¹³C NMR (75.5 MHz, CDCl₃): δ = 140.1 (Cq); 136.4 (Cq); 135.4 (Cq); 134.8; 134.3 (Cq); 133.3 (Cq); 131.9 (Cq); 130.0 (Cq); 128.7; 128.3; 127.5; 125.4; 125.4; 124.2; 123.7 (Cq); 123.2; 122.6; 121.8 (Cq); 118.0; 110.8; 72.3 (CH₂); 55.8 (CH₂); 52.5 (CH₂); 47.3 (CH₂); 11.6; 10.3 ppm.. MS (EI, 70 eV) m/z (rel. intensity): 547 (M⁺, 1), 448 (1), 447 (1), 351 (1), 350 (1), 252 (5), 251 (5), 250 (4), 161 (4), 134 (16), 133 (3), 101 (8), 100 (100), 99 (8), 71 (25), 70 (15), 57 (41), 56 (16), 55 (29). HRMS Calcd. for C₃₀H₃₃N₃O₃S_{2:} 547.19578. Found: 547.197375. FTIR (ATR): 3308, 3106, 2966, 2928, 2851, 1492, 1454, 1346, 1291, 1259, 1233, 1146, 1086, 1063, 1024, 969, 844, 803, 757, 732, 699 cm⁻¹.

### 6-Chloro-imidazo[2,1-b]thiazole-5-sulfonic acid [3-(2-diethylamino-ethoxy)-2-methyl-1H-indol-5-yl]-amide (6b)

In a round bottomed flask under an argon atmosphere 5-(6-chloro-imidazo[2,1-*b*]thiazole-5-sulfonylamino)-3-(2-diethylamino-ethoxy)-2-methyl-indole-1-carboxylic acid *tert*-butyl ester **5h** was heated up to 140 °C for 3 h. After column chromatography (eluent: ethanol gradient 10-30% in CH₂Cl₂) a light cream powder was obtained in 88 % yield.

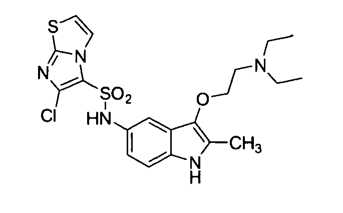

¹H NMR (300.13 MHz, acetone-*d*₆, referenced to solvent signal δ= 2.05 ppm): □= 9.67 (br, 1H, NH); 7.71 (d, 1H, *J* = 4.5 Hz); 7.35 (d, 1H, *J* = 4.5 Hz); 7.30 (d, 1H, *J* = 2.1 Hz); 7.12 (dd, 1H, *J* = 8.5 Hz, *J* = 0.5 Hz); 6.87 (dd, 1H, *J* = 8.5 Hz, *J* = 2.1 Hz); 3.96 (t, 2H, *J* = 6.3 Hz); 2.78 (t, 2H, *J* = 6.3 Hz); 2.61 (q, 4H, *J* = 7.1 Hz); 2.30 (s, 3H); 1.02 (t, 6H, *J* = 7.1 Hz) ppm. ¹³C NMR (75.5 MHz, acetone-*d*₆, referenced to solvent signal δ = 29.9 ppm): δ = 150.4 (Cq); 138.1 (Cq); 135.8 (Cq); 132.5 (Cq); 128.0 (Cq); 125.4 (Cq); 122.8 (Cq); 121.2; 119.7 (Cq); 118.6; 115.9; 113.2; 112.2; 73.6 (CH₂); 53.7 (CH₂); 47.4 (CH₂); 12.6; 10.3 ppm. MS (EI, 70 eV) *m*/*z* (rel. intensity): 481 (M⁺, 0.3), 382 (1), 174 (1), 173 (1), 161 (5) 160 (5), 145 (2), 101 (7), 100 (100), 86 (48), 72 (7), 58 (4). HRMS (ESI⁺, [M+H]⁺) Calcd. for C₂₀H₂₄ClN₅O₃S₂: 482.10819. Found: 482.10949. FTIR (ATR): 3308, 3129, 3059, 2964, 2923, 2853, 1459, 1435, 1269, 1240, 1211, 1177, 1139, 1122, 1103, 1042, 1022, 926, 882, 814, 791, 727, 671 cm⁻¹.

## Claims

1. An indole sulfonamide compound of general formula (I), 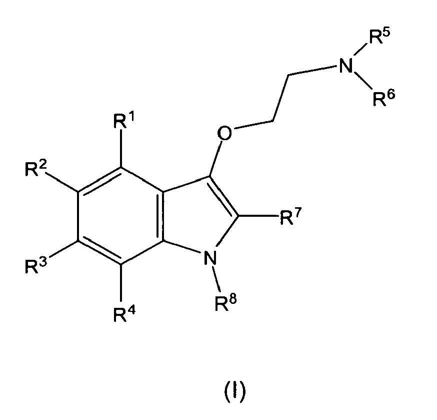 R¹, R², R³, and R⁴ independent from one another, each represents a hydrogen atom, an halogen atom, a C₁₋C₆ alkyl radical, a C₁₋C₆ alkoxy radical or a -N(R⁹)-S(=O)₂-R¹⁰ moiety
with the proviso that at least one of the substituents R¹, R², R³ and R⁴ represents a - N(R⁹)-S(=O)₂-R¹⁰ moiety
wherein
R⁹ represents a hydrogen atom or C₁₋₁₀ alkyl radical optionally substituted with one or more substituents independently selected from C₁₋C₆ alkyl, aryl, cyano, C₁₋C₆ alkoxy and trifluoromethyl;
R¹⁰ represents aryl or heteroaryl optionally substituted with one or more substituents independently selected from -NO₂, -NH₂, -SH, -OH, -CN, -C(=O)-OH, -S(=O)₂-OH, -C(=O)-NH₂, -S(=O)₂-NH₂, -S(=O)₂-R¹¹ , -OR¹¹ , -SR¹¹ , -C(=O)-OR¹¹ , -N(R¹¹)-S(=O)₂-R¹² , -NH-R¹¹ , -NR¹¹R¹² , -C(=O)-NHR¹¹, -C(=O)-NR¹¹R¹² , -S(=O)₂-NHR¹¹, -S(=O)₂-NR¹¹R¹² , -O-C(=O)-R¹¹ , -NH-C(=O)-R¹¹ , -NR¹¹-C(=O)-R¹² , -NH-C(=O)-O-R¹¹ , -NR¹¹-C(=O)-O-R¹² , -S(=O)₂-O-R¹¹ , an halogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene, or an optionally at least mono-substituted aryl or heteroaryl radical, which may be bonded via a linear or branched alkylene,
wherein R¹¹ and R¹², independent from one another, each represents a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene, alkenylene or alkinylene group,
R⁵ and R⁶, independent from one another, each represents a hydrogen atom; or a linear or branched, saturated or unsaturated, unsubstituted or at least mono-substituted aliphatic radical;
or
R⁵ and R⁶ together with the bridging nitrogen atom form a saturated or unsaturated, unsubstituted or at least mono-substituted, optionally at least one additional heteroatom as ring member containing heterocycloaliphatic radical, which may be condensed with an unsubstituted or at least mono-substituted saturated, unsaturated or aromatic mono- or bicyclic ring system;
or
R⁵ and R⁶ together with the bridging nitrogen atom form an unsubstituted or at least mono-substituted, optionally at least one additional heteroatom as ring member containing heteroaryl radical, which may be condensed with an unsubstituted or at least mono-substituted saturated, unsaturated or aromatic mono- or bicyclic ring system;
R⁷ represents a linear or branched, saturated or unsaturated, unsubstituted or at least mono-substituted aliphatic radical;
R⁸ represents a hydrogen atom; a -C(=O)-OR¹³ moiety; a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁-₅-alkyl, -S-C₁₋₅-alkyl, -NH(C₁₋₅-alkyl) and -N(C₁₋₅-alkyl)₂; or a benzyl moiety which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)_{2;}
R¹³ represents a linear or branched, saturated or unsaturated C₁₋₁₀ aliphatic radical
optionally in form of one of its stereoisomers, preferably enantiomers or diasteromers, its racemate or in the form of a mixture of at least two of its stereoisomers, preferably enantiomers or diastereomers, in any mixture ratio, or a salt, preferably a corresponding physiologically acceptable salt, or a prodrug, or a corresponding solvate.

2. An indole sulfonamide compound according to claim 1, wherein R² represents the -N(R⁹)-S(=O)₂-R¹⁰ moiety and R¹, R³, and R⁴ are hydrogen.

3. An indole sulfonamide compound according to claim 1 or 2, wherein R⁹ is selected from hydrogen, C₁₋₁₀ alkyl and a benzyl group.

4. An indole sulfonamide compound according to any of the preceding claims, wherein R¹⁰ represents an heteroaryl radical, which is a 8- or 9- membered aromatic bicyclic system, having from 1 to 3 heteroatoms independently selected from nitrogen and sulphur atoms, substituted with one or more halogen atoms, preferably chloro, and/or with a linear saturated aliphatic radical, preferably methyl.

5. An indole sulfonamide compound according to any of the preceding claims, wherein R⁷ is a saturated aliphatic radical, preferably methyl.

6. An indole sulfonamide compound according to any of the preceding claims, wherein R⁸ is selected from hydrogen, a -C(=O)-OR¹³ moiety, a saturated C₁₋₁₀ aliphatic radical; preferably methyl and benzyl, wherein R¹³ is a branched saturated C₁₋₁₀ aliphatic radical, preferably t-butyl.

7. An indole sulfonamide compound according to any of the preceding claims, wherein R⁵ and R⁶, independent from one another, each represents a saturated C₁₋₁₀ alkyl, radical, preferably ethyl.

8. An indole sulphonamide compound according to any of the preceding claims, selected from the group consisting of:
**(1)** 2,1,3-Benzothiadiazole-5-sulfonic acid [3-(2-diethylamino-ethoxy)-1,2-dimethyl-1*H*-indol-5-yl]-amide **(5a)**
**(2)** 2,1,3-Benzothiadiazole-4-sulfonic acid [1-benzyl-3-(2-diethyl-aminoethoxy)-2-methyl-1*H*-indol-5-yl]-amide **(5b)**
**(3)** 5-[(2,1,3-Benzothiadiazole-4-sulfonyl)-hexyl-amino]-3-(2-dieth-ylamino-ethoxy)-2-methyl-indole-1-carboxylic acid *tert*-butyl ester **(5c)**
**(4)** 5-Chloro-3-methyl-benzo[*b*]thiophene-2-sulfonic acid [3-(2-diethylamino-ethoxy)-1,2-dimethyl-1*H*-indol-5-yl]-amide **(5d)**
**(5)** 5-[(6-Chloro-imidazo[2,1-*b*]thiazole-5-sulfonyl)-hexyl-amino]-3-(2-diethylamino-ethoxy)-2-methyl-indole-1-carboxylic acid *tert*-butyl ester **(5e)**
**(6)** Benzo[*b*]thiophene-2-sulfonic acid [3-(2-diethylamino-ethoxy)-1,2-dimethyl-1*H-*indol-5-yl]-amide **(5f)**
**(7)** 5-[(Benzo[*b*]thiophene-2-sulfonyl)-benzyl-amino]-3-(2-diethyl-amino-ethoxy)-2-methyl-indole-1-carboxylic acid *tert*-butyl ester **(5g)**
**(8)** 5-(6-Chloro-imidazo[2,1-*b*]thiazole-5-sulfonylamino)-3-(2-diethylamino-ethoxy)-2-methyl-indole-1-carboxylic acid tert-butyl ester **(5h)**
**(9)** Benzo[*b*]thiophene-3-sulfonic acid benzyl-[3-(2-diethylamino-ethoxy)-2-methyl-1*H*-indol-5-yl]-amide **(6a)**
**(10)** 6-Chloro-imidazo[2,1-b]thiazole-5-sulfonic acid [3-(2-diethylamino-ethoxy)-2-methyl-1 H-indol-5-yl]-amide **(6b)**

9. A process for the preparation of an indole sulfonamide compound according to one or more of claims 1 to 8, which comprises reacting at least one compound of formula **3** or **4** 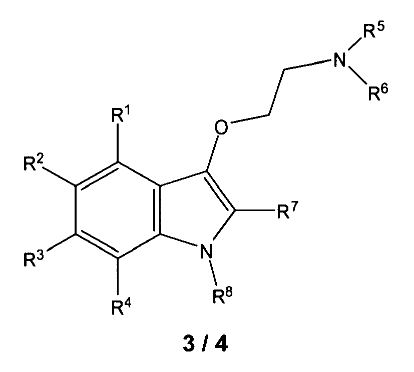 wherein R¹, R², R³, R⁴, R⁵, R⁶, R⁷ and R⁸ have the meaning according to one or more of claims 1 to 8, with the proviso that at least one of R¹, R², R³, R⁴ is -NHR⁹ in compound of formula **3,** or -NH₂ in compound of formula **4,** with at least one compound of general formula **(II),**
**R¹⁰SO₂X** **II ,**
wherein R¹⁰ has the respective meaning according to one or more of claims 1 to 8, and **X** represents a leaving group preferably selected from the group consisting of Cl and Br, optionally in the presence of at least one base, preferably in the presence of at least one inorganic base selected from the group consisting of alkali metal hydroxides or carbonates or in the presence of at least one organic base selected from the group consisting of triethylamine or pyridine, at temperatures from 0°C to room temperature and with reaction time from 5 minutes to 24 hours to yield at least one compound of general formula (I), 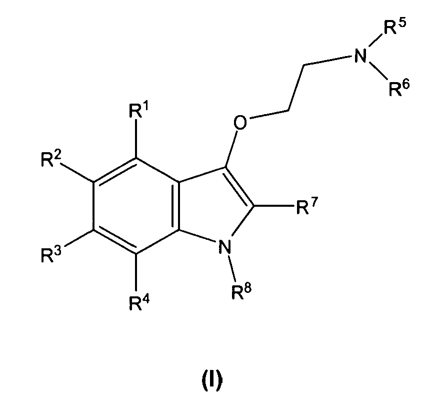 wherein R¹, R², R³, R⁴, R⁵, R⁶, R⁷ and R⁸ have the meaning according to one or more of claims 1 to 8.

10. Process according to claim 9, which further comprises the following steps:
**a)** deprotecting at least one compound of formula **(1)** 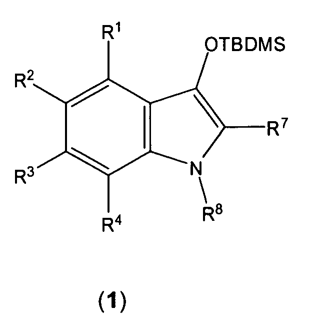 wherein R¹, R², R³, R⁴, R⁷ and R⁸ have the meaning according to one or more of claims 1 to 8, with the proviso that at least one of R¹, R², R³, R⁴ is Br, with a desilylating agent as TBAF at room temperature in a reaction medium, as THF, followed by condensation with at least one compound of general formula **(III)**
**R⁵R⁶NCH₂CH₂X** **III**
wherein R⁵ and R⁶ have the meaning according to one or more of claims 1 to 8 and **X** represents a leaving group preferably selected from the group consisting of Cl and Br, at temperatures from 25 to 100 °C with reaction time from 3 to 12 hours, to yield at least one compound of formula **2** 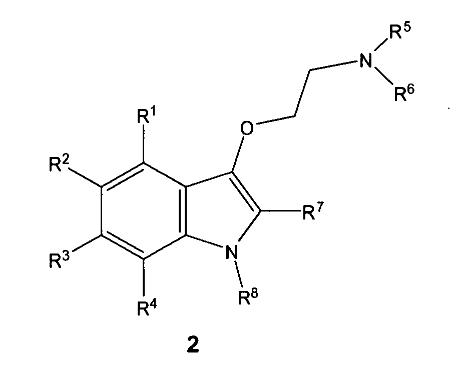 wherein R¹, R², R³, R⁴, R⁵ and R⁶, R⁷ and R⁸ have the meaning according to one or more of claims 1 to 8;
**b)** palladium-catalyzed amination of at least a compound of formula **2** with a compound of formula **(IV)**
**NH₂R⁹** **(IV)**
to yield at least a compound of formula **3** wherein R⁹ has the meaning according to one or more of claims 1 to 8; and
**c)** optionally, catalyzed hydrogenation of at least a compound of formula **3** to yield at least a compound of formula **4.**

11. A compound of one of the following formulae **3** or **4** 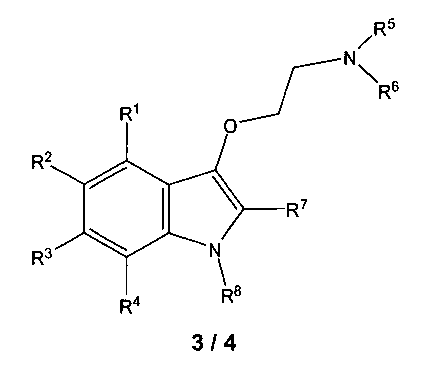 wherein R¹, R², R³, R⁴, R⁵, R⁶, R⁷ and R⁸ have the meaning according to one or more of claims 1 to 8, with the proviso that at least one of R¹, R², R³, R⁴ is -NHR⁹ in compound of formula **3,** or -NH₂ in compound of formula **4,** and wherein R⁹ has the meaning according to one or more of claims 1 to 8.

12. A compound according to claim 11, selected from the group consisting of:
Benzyl-[3-(2-diethylamino-ethoxy)-1,2-dimethyl-1*H*-indol-5-yl]-amine **(3a)**
Benzyl-[1-benzyl-3-(2-diethylamino-ethoxy)-2-methyl-1*H*-indol-5-yl]-amine **(3b)**
5-Benzylamino-3-(2-diethylamino-ethoxy)-2-methyl-indole-1-carboxylicacid *tert-*butyl ester **(3c)**
[3-(2-Diethylamino-ethoxy)-1,2-dimethyl-1*H*-indol-5-yl]-hexyl-amine **(3d)**
3-(2-Diethylamino-ethoxy)-5-hexylamino-2-methyl-indole-1-carboxylic acid *tert*-butyl ester **(3e)**
[3-(2-Diethylamino-ethoxy)-1,2-dimethyl-1*H*-indol-5-yl]-[2-(4-fluoro-phenyl)-ethyl]-amine **(3f)**
3-(2-Diethylamino-ethoxy)-1,2-dimethyl-1*H*-indol-5-ylamine **(4a)**
1-Benzyl-3-(2-diethylamino-ethoxy)-2-methyl-1*H-*indol-5-ylamine **(4b)**
5-Amino-3-(2-diethylamino-ethoxy)-2-methyl-indole-l-carboxylic acid *tert*-butyl ester **(4c).**

13. A medicament comprising at least one indole sulphonamide compound according to one or more of claims 1 to 8, and at least a pharmaceutical acceptable carrier, adjuvant or vehicle.

14. A medicament according to claim 13, for the treatment and/or prophylaxis of diseases or disorders that are at least partially mediated via 5-HT₆ receptors.

15. A medicament according to claim 13 or 14, for the prophylaxis and/or treatment of food intake disorders, preferably for the regulation of appetite, for the maintenance, increase or reduction of body weight, for the prophylaxis and/or treatment of obesity, for the prophylaxis and/or treatment of bulimia, for the prophylaxis and/or treatment of anorexia, for the prophylaxis and/or treatment of cachexia, for the prophylaxis and/or treatment of type II diabetes (non-insulin dependent diabetes mellitus); for the prophylaxis and/or treatment of cognitive disorders, preferably memory disorders; or for improvement of cognition (for cognitive enhancement).

16. A medicament according to claim 13 or 14, for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia or for the prophylaxis and/or treatment hyperactivity disorder (ADHD, attention deficit, hyperactivity disorder).

17. Use of at least one indole sulfonamide compound according to one or more of claims 1 to 8, for the manufacture of a medicament for regulating the 5-HT₆ receptor.

18. Use of at least one indole sulfonamide compound according to any one of claims 1 to 8, for the manufacture of a medicament for the prophylaxis and/or treatment of disorders that are at least partially mediated via 5-HT₆ receptors.

19. Use of at least one indole sulfonamide compound according to any one of claims 1 to 8, for the prophylaxis and/or treatment of food intake disorders; for the prophylaxis and/or treatment of cognitive disorders; or for improvement of cognition (for cognitive enhancement).

20. Use of at least one indole sulfonamide compound according to claim 19, for the regulation of appetite, for the reduction, increase or maintenance of body weight; for the prophylaxis and/or treatment of obesity, for the prophylaxis and/or treatment of bulimia, for the prophylaxis and/or treatment of anorexia; for the prophylaxis and/or treatment of cachexia; for the prophylaxis and/or treatment of type II diabetes; or for the prophylaxis and/or treatment of memory disorders.

21. Use of at least one indole sulfonamide compound according to any one of claims 1 to 8 for the manufacture of a medicament for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia; or for the prophylaxis and/or treatment hyperactivity disorder (ADHD, attention deficit, hyperactivity disorder).
